# EUROPEAN PATENT APPLICATION

(11) **EP 4 194 482 A1**
(43) Date of publication of application: **14.06.2023**
(21) Application number: 21837851.1
(22) Date of filing: 01.07.2021
(51) Int. Cl.: C08G 73/22, B32B 27/00, B32B 27/18, B32B 27/34, C07C 215/80, H01B 3/30, H05K 1/03

(54) **POLYBENZOXAZOLE, POLYAMIDE, POLYAMIDE SOLUTION, INSULATION FOR HIGH-FREQUENCY ELECTRONIC COMPONENT, HIGH-FREQUENCY ELECTRONIC COMPONENT, HIGH-FREQUENCY EQUIPMENT, INSULATION MATERIAL FOR PRODUCING HIGH-FREQUENCY ELECTRONIC COMPONENT, METHOD FOR PRODUCING POLYAMIDE, METHOD FOR PRODUCING POLYBENZOXAZOLE, METHOD FOR PRODUCING INSULATION FOR HIGH-FREQUENCY ELECTRONIC COMPONENT, AND DIAMINE OR SALT THEREOF**

(30) Priority: 10.07.2020 JP 2020119001
(71) Applicant: Central Glass Company, Limited, Ube-shi, Yamaguchi 755-0001 (JP)
(72) Inventor: MURAKAMI Yosuke, Tokyo 101-0054 (JP); HOSOI Kenji, Tokyo 101-0054 (JP); EGUCHI Hiroshi, Tokyo 101-0054 (JP); HAGIWARA Yuki, Tokyo 101-0054 (JP); YAMADA Masaki, Tokyo 101-0054 (JP); YAMANAKA Kazuhiro, Tokyo 101-0054 (JP)
(74) Representative: Manitz Finsterwald Patent- und Rechtsanwaltspartnerschaft mbB
(86) International application number: PCT/JP2021/025005
(87) International publication number: WO 2022/009782

(57) **Abstract**

Provided is a polybenzoxazole having a structural unit represented by General Formula [1]. In General Formula [1], R¹ is a tetravalent organic group represented by General Formula [2], and R² is a divalent organic group. In General Formula [2], two n's are each independently an integer of 0 to 3, in a case where a plurality of R³' s are present, the plurality of R³' s each independently represent a monovalent substituent, and *1, *2, *3, and *4 each independently represent a bonding site, in which one of *1 and *2 is bonded to an oxygen atom in General Formula [1] and the other is bonded to a nitrogen atom in General Formula [1], and one of *3 and *4 is bonded to an oxygen atom in General Formula [1] and the other is bonded to a nitrogen atom in General Formula [1].

## Description

### TECHNICAL FIELD

The present invention relates to a polybenzoxazole, a polyamide, a polyamide solution, an insulating material for a high-frequency electronic component, a high-frequency electronic component, a high-frequency equipment, an c for producing a high-frequency electronic component, a method for producing a polyamide, a method for producing a polybenzoxazole, a method for producing an insulating material for a high-frequency electronic component, and a diamine or a salt thereof.

### BACKGROUND ART

High-frequency electrical signals are used in communication devices (high-frequency equipment) typified by mobile phones and smart phones. In order to speed up the propagation speed of the electrical signals, there is a method of shortening the wiring length by miniaturizing semiconductor elements and increasing the density of mounting boards, but the propagation speed can also be increased by lowering the relative permittivity (εr) of the insulating material forming the mounting board in the high-frequency equipment.

As the frequency of electrical signals is higher, it is easily attenuated, and transmission loss may increase. In order to reduce the transmission loss, a material with a low dielectric loss tangent (tan δ) may be required as an insulating material constituting a mounting board in a high-frequency equipment.

Patent Document 1 discloses a fluorine-containing polyimide film having a dielectric loss tangent of 0.007 or less, a water absorption rate of 0.8% or less, and a linear expansion coefficient at 50°C to 200°C of 30 ppm/°C or less. According to the disclosure of Patent Document 1, the fluorine-containing polyimide film has properties such as low dielectric constant and low water absorption (and low permeability to water vapor and gas), and is particularly suitable for high-frequency substrates.

### RELATED DOCUMENT

### PATENT DOCUMENT

[Patent Document 1] Japanese Unexamined Patent Publication No. 2018-165346

### SUMMARY OF THE INVENTION

### TECHNICAL PROBLEM

With the start of the so-called 5G (fifth generation mobile communication system) service, in the 5G frequency band, an insulating material with a small relative permittivity and a small dielectric loss tangent is more demanded than ever before. For example, from the viewpoint of a low dielectric constant and a low dielectric loss tangent, it is considered that the fluorine-containing resin such as that disclosed in Patent Document 1 is preferable (probably due to peculiar electron-withdrawing properties of the fluorine atom).

On the other hand, in the manufacturing process of semiconductor elements and mounting substrates, a recent trend is to lower the temperature of the heating process. For example, in a case of heating a resin composition to form an insulating material, a low heating temperature is often required. That is, there is a demand for a resin composition for forming an insulating material, which is cured at a relatively low temperature.

Based on the preliminary studies and past knowledge of the present inventors, in a resin composition including the fluorine-containing resin in the related art, there is room for improvement in achieving both a low dielectric constant and a low dielectric loss tangent and curing at a relatively low temperature.

The present invention has been made in view of such circumstances . One object of the present invention is to provide a fluorine-containing resin material which has a low relative permittivity and dielectric loss tangent and can be cured at a relatively low temperature to form an insulating material.

### SOLUTION TO PROBLEM

The present invention is a polybenzoxazole having a structural unit represented by General Formula [1]. 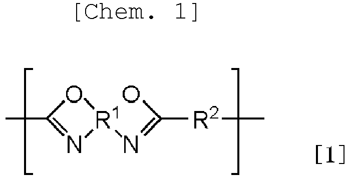 In General Formula [1],
R¹ is a tetravalent organic group represented by General Formula [2], and
R² is a divalent organic group. 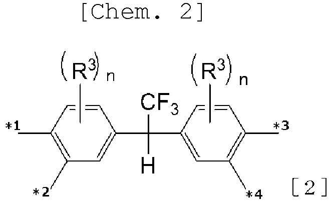 In General Formula [2],
two n's are each independently an integer of 0 to 3,
in a case where a plurality of R³'s are present, the plurality of R³'s each independently represent a monovalent substituent, and
*1, *2, *3, and *4 each independently represent a bonding site, in which one of *1 and *2 is bonded to an oxygen atom in General Formula [1] and the other is bonded to a nitrogen atom in General Formula [1], and one of *3 and *4 is bonded to an oxygen atom in General Formula [1] and the other is bonded to a nitrogen atom in General Formula [1] .

In addition, the present invention is a polyamide having a structural unit represented by General Formula [1A]. 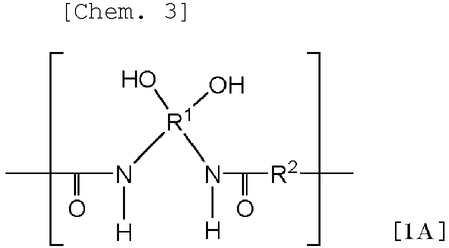 In General Formula [1A],
R¹ represents a tetravalent organic group represented by General Formula [2], and
R² represents a divalent organic group. 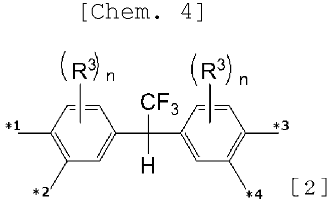 In General Formula [2],
two n's are each independently an integer of 0 to 3,
in a case where a plurality of R³'s are present, the plurality of R³'s each independently represent a monovalent substituent, and
*1, *2, *3, and *4 each independently represent a bonding site, in which one of *1 and *2 is bonded to an oxygen atom in a hydroxy group of General Formula [1A] and the other is bonded to a nitrogen atom in General Formula [1A], and one of *3 and *4 is bonded to an oxygen atom in a hydroxy group of General Formula [1A] and the other is bonded to a nitrogen atom in General Formula [1A].

In addition, the present invention is a polyamide solution containing the above-described polyamide and an organic solvent.

In addition, the present invention is an insulating material for a high-frequency electronic component, including the above-described polybenzoxazole.

In addition, the present invention is a high-frequency electronic component including the above-described insulating material for a high-frequency electronic component.

In addition, the present invention is a high-frequency equipment including the above-described high-frequency electronic component.

In addition, the present invention is an insulating material for producing a high-frequency electronic component, including the above-described polyamide.

In addition, the present invention is a method for producing the above-described polyamide, the method including a step of polycondensing a diamine represented by General Formula [DA] or a salt of the diamine with a dicarboxylic acid represented by General Formula [DC1] or [DC2] or a derivative of the dicarboxylic acid.

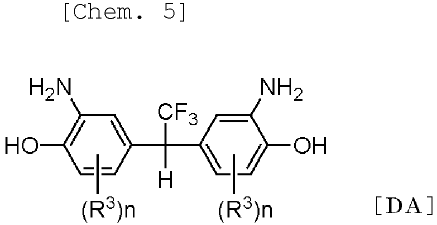

In General Formula [DA], n and R³ are the same as n and R³ in General Formula [2].

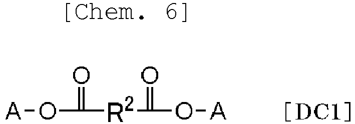

In General Formula [DC1], R² is the same as R² in General Formula [1A], and two A's are each independently a hydrogen atom, an alkyl group having 1 to 10 carbon atoms, or an aromatic hydrocarbon group having 6 to 10 carbon atoms.

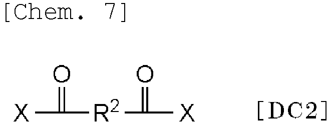

In General Formula [DC2], R² is the same as R² in General Formula [1A], and two X's are each independently a fluorine atom, a chlorine atom, a bromine atom, an iodine atom, or an active ester group.

In addition, the present invention is a method for producing the above-described polybenzoxazole, the method including a first step of producing a polyamide by the above-described method for producing the polyamide and a second step of dehydrating and ring-closing the polyamide obtained in the first step.

In addition, the present invention is a method for producing an insulating material for a high-frequency electronic component, the method including a coating step of applying the above-described polyamide solution onto a supporting base material, a drying step of drying the solvent contained in the applied polyamide solution to obtain a resin film including the polyamide, and a heating step of heat-treating the resin film to form a cured film.

In addition, the present invention is a diamine represented by General Formula [DA] or a salt of the diamine.

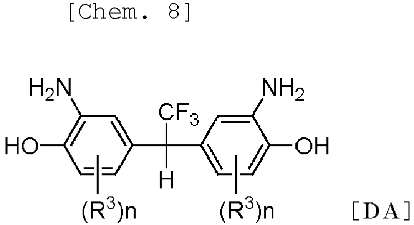

In General Formula [DA], two n's are each independently an integer of 0 to 3, and in a case where a plurality of R³' s are present, the plurality of R³'s each independently represent a monovalent substituent.

In addition, the present invention is a method for producing the above-described diamine or the salt of the diamine, the method including a step of reducing a bisnitrophenol represented by General Formula [DN].

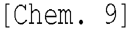

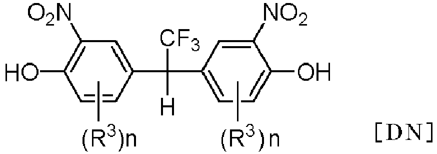

In General Formula [DN], definitions of two n's and R³ are the same as definitions of two n's and R³ in General Formula [DA].

### ADVANTAGEOUS EFFECTS OF INVENTION

According to the present invention, a fluorine-containing resin material, which has a low relative permittivity and dielectric loss tangent and can be cured at a relatively low temperature to form an insulating material, is provided.

### DESCRIPTION OF EMBODIMENTS

Hereinafter, embodiments of the present invention will be described in detail.

In the present specification, the notation "X to Y" in the description of numerical range means X or more and Y or less, unless otherwise specified. For example, "1% to 5% by mass" means "1% by mass or more and 5% by mass or less".

In the notation of groups (atomic groups) in the present specification, a notation without indicating whether it is substituted or unsubstituted includes both those having no substituent and those having a substituent. For example, a term "alkyl group" includes not only alkyl groups having no substituent (unsubstituted alkyl groups) but also alkyl groups having a substituent (substituted alkyl groups).

A term "organic group" used in the present specification means an atomic group obtained by removing one or more hydrogen atoms from an organic compound, unless otherwise specified. For example, a "monovalent organic group" represents an atomic group obtained by removing one hydrogen atom from an optional organic compound.

In the chemical formulae in the present specification, a notation "Me" represents a methyl group (CH₃).

In the present specification, a term "fluoral" means trifluoroacetaldehyde.

A term "high frequency" in the present specification means, for example, a frequency range of 1 GHz or more, preferably 10 to 200 GHz and more preferably 28 to 100 GHz.

### <Polybenzoxazole>

A polybenzoxazole according to the present embodiment has a structural unit represented by General Formula [1].

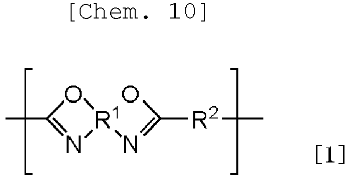

In General Formula [1], R¹ represents a tetravalent organic group represented by General Formula [2], and R² represents a divalent organic group.

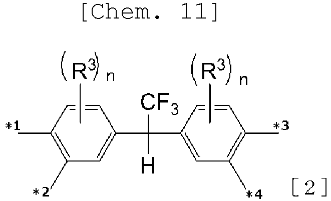

In General Formula [2], two n's are each independently an integer of 0 to 3, and in a case where a plurality of R³'s are present, the plurality of R³'s are each independently a monovalent substituent. In addition, *1, *2, *3, and *4 each independently represent a bonding site, in which one of *1 and *2 is bonded to an oxygen atom in General Formula [1] and the other is bonded to a nitrogen atom in General Formula [1], and one of *3 and *4 is bonded to an oxygen atom in General Formula [1] and the other is bonded to a nitrogen atom in General Formula [1].

The polybenzoxazole according to the present embodiment includes a fluorine atom. In addition to the presence of the polybenzoxazole skeleton, probably due to the presence of the fluorine atom (fluorine atom is present in a -CH(CF₃)- moiety in General Formula [2]), it is presumed that relative permittivity and dielectric loss tangent are small.

In addition, probably due to its rigid cyclic skeleton, the polybenzoxazole according to the present embodiment has high heat resistance.

The polybenzoxazole according to the present embodiment can typically be obtained by heating a polyamide having a structural unit represented by General Formula [1A] shown later to cause a ring closure reaction. Here, the polyamide having a structural unit represented by General Formula [1A] undergoes the ring closure reaction at a relatively low temperature and is converted to the polybenzoxazole having the structural unit represented by General Formula [1]. The reason for this is not necessarily clear, but it is presumed that the structure "-CH(CF₃)-" is probably involved.

In a case where fluorine is introduced into polybenzoxazole, from the viewpoint of price and availability of raw materials, it is considered common to those skilled in the art to employ a "-C(CF₃)₂-" structure. Compared to the "-C(CF₃)₂-" structure, since the "-CH(CF₃)-" structure of the present embodiment is less symmetrical (more asymmetrical), it is considered that more flexible polymer chains are obtained and allow ring closure reactions at lower temperatures. In addition, the increased asymmetry leads to looser packing of the polymer chains, and as a result, it is considered to lead to a lower polymer density and a lower dielectric constant.

That is, the "-CH(CF₃)-" structure has fewer fluorine atoms than the "-C(CF₃)₂-" structure, which is considered common to those skilled in the art, but due to the looser packing of the polymer chains, it is presumed that the structure has excellent low-temperature curability while exhibiting substantially the same dielectric properties.

In General Formula [2], the monovalent substituent for R³ is not particularly limited. Examples thereof include an alkyl group, an alkoxy group, a cycloalkyl group, an aryl group, an alkenyl group, an alkynyl group, an aryloxy group, an amino group, an alkylamino group, an arylamino group, a cyano group, a nitro group, a silyl group, and a halogeno group (for example, a fluoro group) . These may further have a substituent such as a fluorine atom and a carboxyl group.

As the monovalent substituent of R³, an alkyl group, an alkoxy group, a fluorinated alkyl group (for example, a trifluoromethyl group), a halogeno group (for example, a fluoro group), or a nitro group is preferable.

Specific examples of the alkyl group in R³ include linear or branched alkyl groups having 1 to 6 carbon atoms. Among these, an n-butyl group, an s-butyl group, an isobutyl group, a t-butyl group, an n-propyl group, an i-propyl group, an ethyl group, or a methyl group is preferable, and an ethyl group or a methyl group is more preferable.

Specific examples of the alkoxy group in R³ include linear or branched alkoxy groups having 1 to 6 carbon atoms. Among these, an n-butoxy group, an s-butoxy group, an isobutoxy group, a t-butoxy group, an n-propoxy group, an i-propoxy group, an ethoxy group, or a methoxy group is preferable, and an ethoxy group or a methoxy group is particularly preferable.

The alkyl group or the alkoxy group in R³ may be substituted on any carbon thereof with any number and any combination of, for example, halogen atoms, alkoxy groups, and haloalkoxy groups. Furthermore, in a case where the number of R³'s is 2 or more, these two or more R³'s may be linked to each other to form a saturated or unsaturated, monocyclic or polycyclic group having 3 to 10 carbon atoms.

In General Formula [2], n is preferably 0 to 2, more preferably 0 or 1, and still more preferably 0.

Particularly preferred examples of R¹ include the following. In the following, the methyl group is specified as Me to distinguish it from a bonding site.

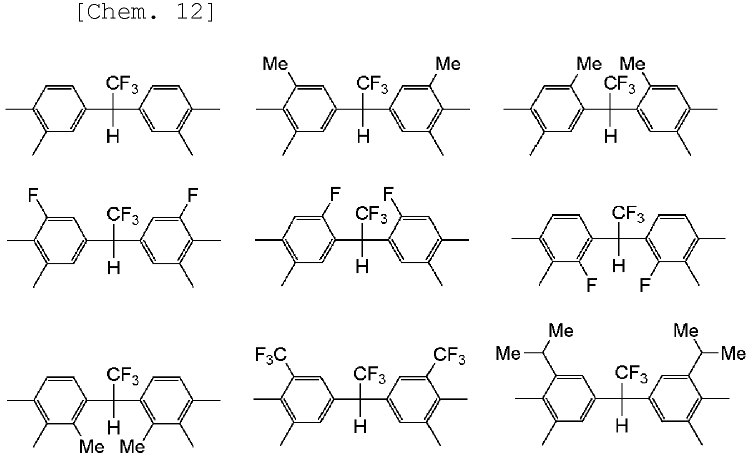

In General Formula [1], the divalent organic group for R² is not particularly limited. From the viewpoint of heat resistance required for insulating material and better dielectric properties, a divalent organic group containing an aromatic ring such as a benzene ring is preferable. More specifically, the divalent organic group for R² can be -Ph-, -Ph-X-Ph-, or the like. Here, Ph is a substituted or unsubstituted phenylene group, X is a single bond or a divalent linking group other than the phenylene group (for example, a linear or branched alkylene group having 1 to 3 carbon atoms, an ether group, a thioether group, a carbonyl group, a sulfone group, a carbonyloxy group, an oxycarbonyl group, and the like).

Particularly preferred examples of R² include the following.

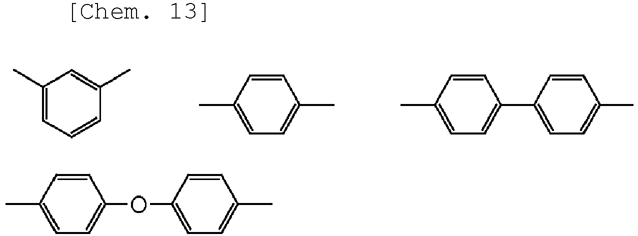

Preferred examples of the structural unit represented by General Formula [1] are shown below.

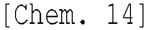

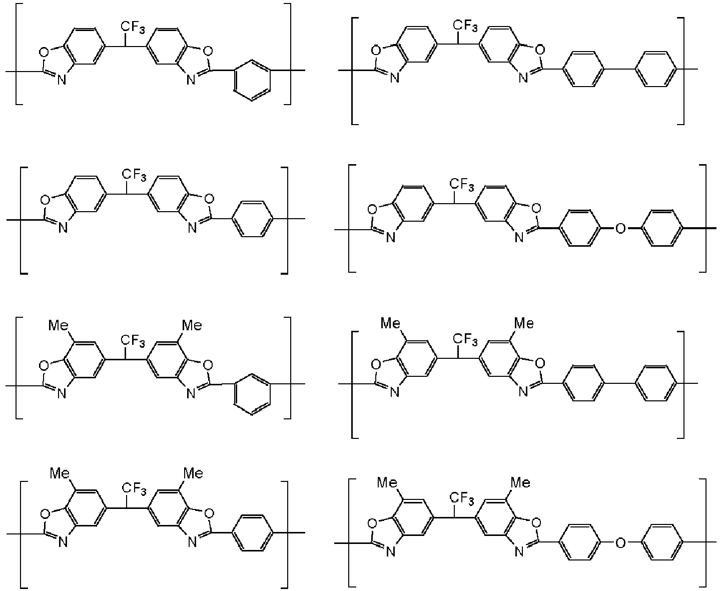

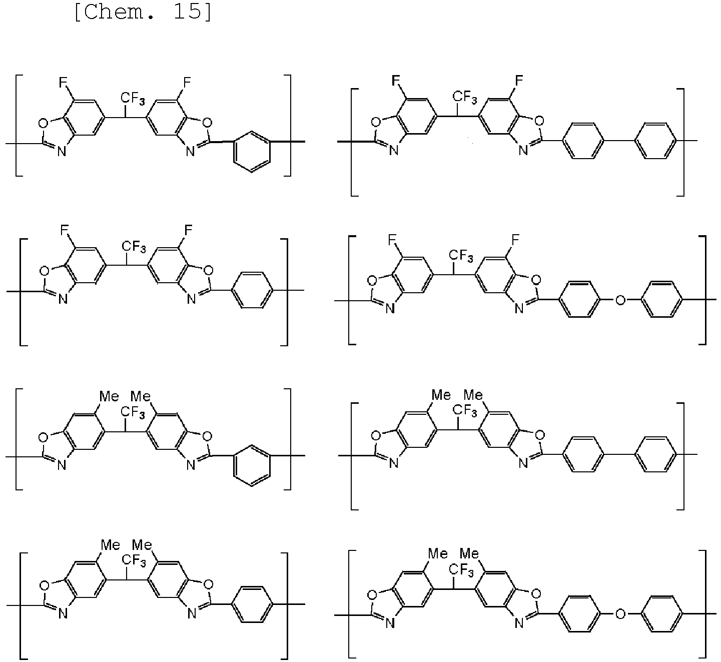

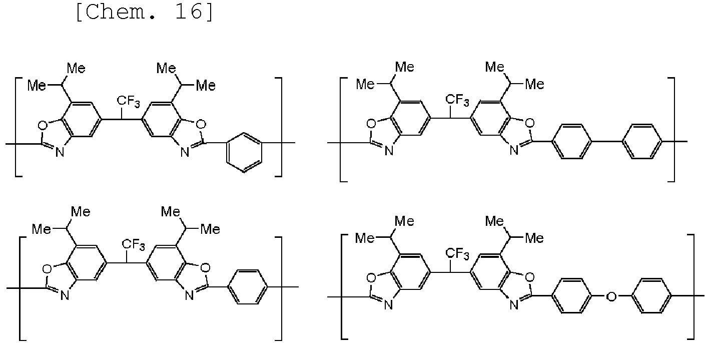

A weight-average molecular weight, number-average molecular weight, and the like of the polybenzoxazole according to the present embodiment are generally comparable to those of a polyamide described later.

The polybenzoxazole according to the present embodiment may have a structural unit different from the structural unit represented by General Formula [1]. However, in order to particularly reduce the relative permittivity and dielectric loss tangent and/or to form an insulating material by curing at a sufficiently low temperature, in all structural units of the polybenzoxazole, preferably 50 to 100 mol%, more preferably 75 to 100 mol%, and still more preferably 90 to 100 mol% are the structural unit represented by General Formula [1]. Substantially all structural units (100%) of the polybenzoxazole according to the present embodiment may be the structural unit represented by General Formula [1].

### <Polyamide/insulating material for producing a high-frequency electronic component>

A polyamide (polyhydroxyamide) according to the present embodiment has a structural unit represented by General Formula [1A].

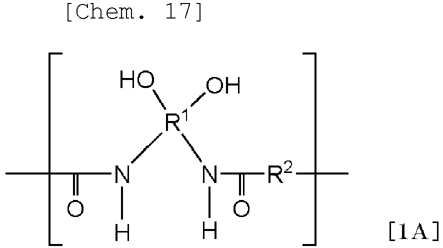

In General Formula [1A], R¹ represents a tetravalent organic group represented by General Formula [2], and R² represents a divalent organic group.

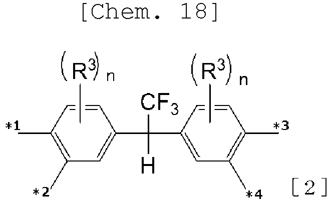

In General Formula [2], two n's are each independently an integer of 0 to 3, and in a case where a plurality of R³'s are present, the plurality of R³'s each independently represent a monovalent substituent. In addition, *1, *2, *3, and *4 each independently represent a bonding site, in which one of *1 and *2 is bonded to an oxygen atom in a hydroxy group of General Formula [1A] and the other is bonded to a nitrogen atom in General Formula [1A], and one of *3 and *4 is bonded to an oxygen atom in a hydroxy group of General Formula [1A] and the other is bonded to a nitrogen atom in General Formula [1A].

Specific aspects of the divalent organic group for R² in General Formula [1A] are the same as in General Formula [1]. In addition, the structure itself represented by General Formula [2] described above and specific aspects of n and R³ are the same as those of General Formula [2] described in the section of <Polybenzoxazole>. Therefore, no further detailed explanation will be given.

Preferred examples of the structural unit represented by General Formula [1A] are shown below.

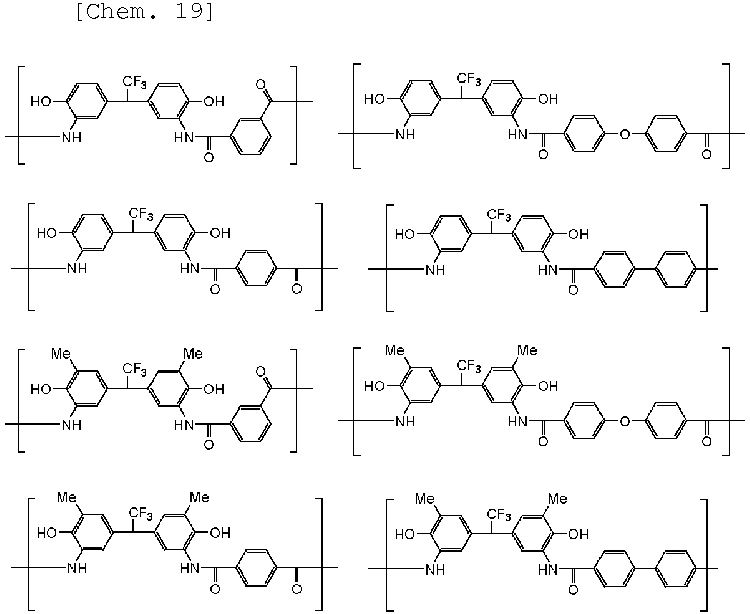

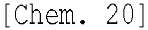

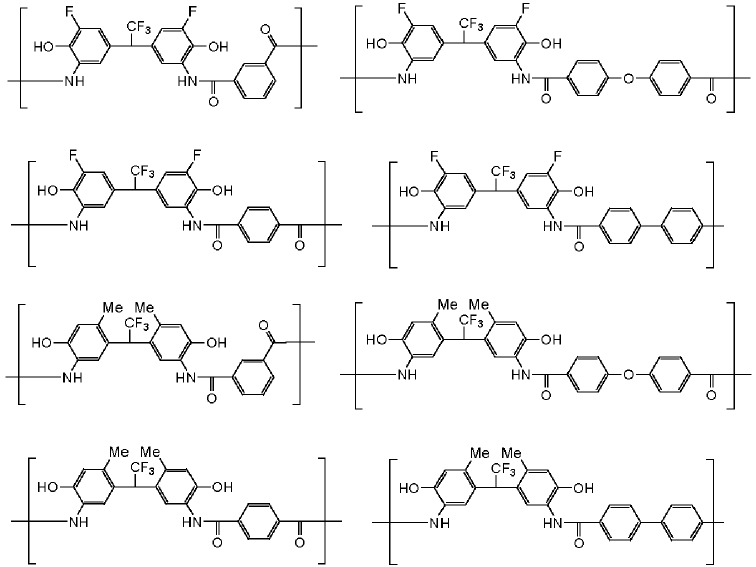

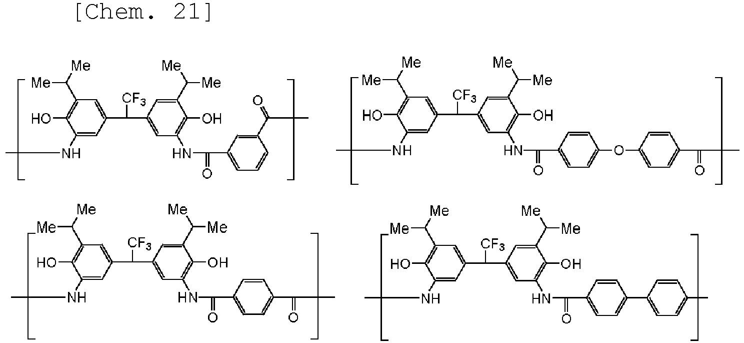

A weight-average molecular weight of the polyamide according to the present embodiment is not particularly limited. However, due to the performance in a case where it is made into a cured film and the ease of forming a film on a base material, the weight-average molecular weight of the polyamide is preferably 1,000 or more and 1,000,000 or less, and more preferably 30,000 or more and 500,000 or less. Particularly in consideration of application to high-frequency electronic component production, the weight-average molecular weight is preferably 500,000 or less. The weight-average molecular weight and number-average molecular weight can be measured by gel permeation chromatography (GPC) using polystyrene as a standard substance.

The polyamide according to the present embodiment may have a structural unit different from the structural unit represented by General Formula [1A]. However, in order to particularly reduce the relative permittivity and dielectric loss tangent of the polybenzoxazole obtained by the ring closure reaction and/or to form an insulating material by curing at a sufficiently low temperature, in all structural units of the polyamide, preferably 50 to 100 mol%, more preferably 75 to 100 mol%, and still more preferably 90 to 100 mol% are the structural unit represented by General Formula [1A]. Substantially all structural units (100%) of the polyamide according to the present embodiment may be the structural unit represented by General Formula [1A].

The polyamide according to the present embodiment is preferably used as an insulating material for producing a high-frequency electronic component. Specific applications will be detailed later.

### <Polyamide solution>

The polyamide according to the present embodiment (having the structural unit represented by General Formula [1A]) is usually dissolved in an organic solvent and applied to various uses. One of the preferably applied uses is high-frequency electronic component production applications. That is, a polyamide solution which contains the polyamide according to the present embodiment and an organic solvent is preferably used as an insulation material for producing a high-frequency electronic component.

The organic solvent is preferably at least one selected from the group consisting of an amide-based solvent, an ether-based solvent, an aromatic solvent, a halogen-based solvent, and a lactone-based solvent. These solvents dissolve the polyamide according to the present embodiment well. Specific examples of these solvents include the same organic solvents as those used in the reaction (polycondensation) described in <Method for producing polyamide> below. Incidentally, in a case of preparing the polyamide solution, from the viewpoint of simplification of the production process, it is preferable to use the same organic solvent as the organic solvent used in the reaction (polycondensation).

A concentration of the polyamide solution may be appropriately set according to the application and purpose. From the viewpoint of good film-forming properties, the concentration of the polyamide is preferably 0.1% by mass or more and 50% by mass or less, and more preferably 1% by mass or more and 30% by mass or less.

The polyamide solution according to the present embodiment may contain one or two or more additives in addition to the polyamide and the organic solvent. For example, for the purpose of improving coating properties, leveling properties, film forming properties, storage stability, defoaming properties, and the like, additives such as a surfactant can be used.

Examples of a commercially available product of the surfactant include product name MEGAFACE manufactured by DIC Corporation, product number F142D, F172, F173, or F183; product name Fluorad manufactured by 3M, product number FC-135, FC-170C, FC-430, or FC-431; product name Surflon manufactured by AGC SEIMI CHEMICAL CO., LTD., product number S-112, S-113, S-131, S-141, or S-145; and product name SH-28PA, SH-190, SH-193, SZ-6032, or SF-8428 manufactured by Dow Corning Toray Silicone Co., Ltd. (MEGAFACE is the product name of fluorine-based additives (surfactants or surface modifiers) manufactured by DIC Corporation, Fluorad is the product name of fluorine-based surfactants manufactured by 3M, and Surflon is the product name of fluorine-based surfactants of AGC SEIMI CHEMICAL CO., LTD., each of which is registered as a trademark).

In a case where a surfactant is used, an amount thereof is usually 0.001 to 10 parts by mass with respect to 100 parts by mass of the polyamide.

Incidentally, the polyamide solution according to the present embodiment usually does not contain a photosensitizer such as a quinonediazide compound, or contain a small amount thereof. Specifically, the amount of the photosensitizer in the polyamide solution according to the present embodiment is, for example, 1 part by mass or less, specifically 0.1 parts by mass or less with respect to 100 parts by mass of the polyamide. In a case where the polyamide solution according to the present embodiment is used for applications which do not require patterning with light, no photosensitizer is required. In other words, the polyamide solution according to the present embodiment can be non-photosensitive.

### <Method for producing polyamide>

The polyamide according to the present embodiment (having the structural unit represented by General Formula [1A]) can typically be produced by a reaction (polycondensation) of a diamine compound (monomer) or a derivative thereof with another compound (monomer). The reaction is usually performed by reacting a diamine compound (monomer) or a derivative thereof with another compound (monomer) in an organic solvent.

Preferred examples of the diamine compound or a derivative thereof include a diamine represented by General Formula [DA] and a salt thereof.

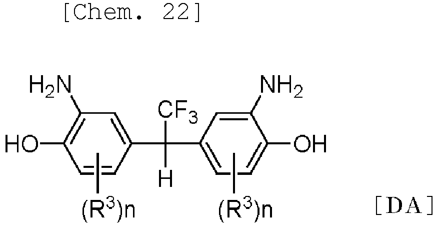

In General Formula [DA], definitions or specific aspects of n and R³ are the same as n and R³ in General Formula [2].

Incidentally, it is considered that the "salt" of the diamine represented by General Formula [DA] is a cationized form of the amino group under acidic conditions, and an anionized form of the phenolic hydroxy group under basic conditions. Just to make sure, both of these two forms are included in the "salt of the diamine represented by General Formula [DA]". Incidentally, the salt of the diamine represented by General Formula [DA] is preferably a cationized form of the amino group.

The diamine represented by General Formula [DA] can be obtained, for example, by reacting (i) 2-aminophenol or a derivative thereof (specifically, 2-aminophenol substituted with the substituent R³ in General Formulae [2] and [DA]) with (ii) fluoral. The molar ratio of reactions (i) and (ii) is usually 2:1.

In addition, the diamine represented by General Formula [DA] may be obtained by reducing the corresponding nitro group-containing compound (the amino group in General Formula [DA] is replaced with a nitro group).

In addition, a salt of the diamine can be obtained by reacting the diamine obtained as described above with an acid.

Examples of specific reaction conditions and fluoral are described later.

Preferred examples of the diamine represented by General Formula [DA] include the following. Needless to say, the diamine which can be used is not limited to these.

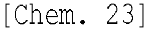

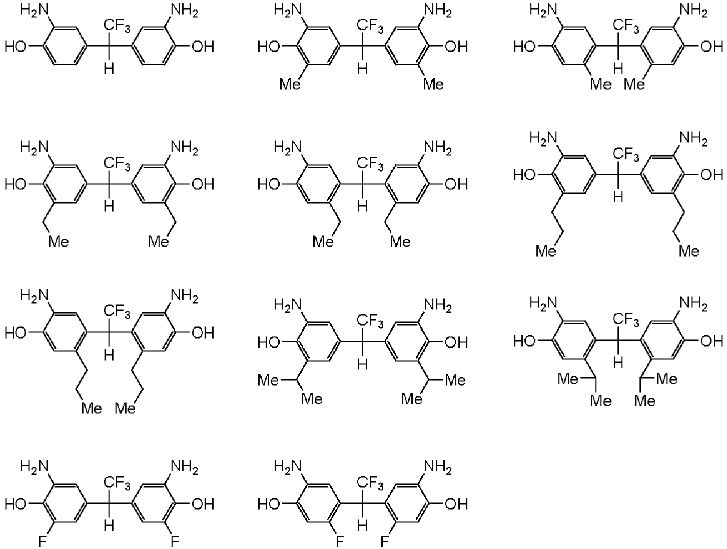

From the viewpoint of cost and performance adjustment, the diamine compound represented by General Formula [DA] and the salt thereof may be used in combination with another diamine compound or a salt thereof. Examples of the diamine compound which can be used include 5-(trifluoromethyl)-1,3-phenylenediamine, 2-(trifluoromethyl)-1,3-phenylenediamine, 4-(trifluoromethyl)-1,3-phenylenediamine, 2-(trifluoromethyl)-1,4-phenylenediamine, 2,2'-bis(trifluoromethyl)benzidine, 2,2'-difluoro-4,4'-diaminodiphenyl, 2,2'-dichloro-4,4'-diaminodiphenyl, 2,2'-dibromo-4,4'-diaminodiphenyl, 2,4-diaminotoluene, 2,5-diaminotoluene, 2,4-dimethyl-1,3-phenylenediamine, 2,5-dimethyl-1,3-phenylenediamine, 2,3-dimethyl-1,4-phenylenediamine, 2,5-dimethyl-1,4-phenylenediamine, 2,6-dimethyl-1,4-phenylenediamine, 2,3,5,6-tetramethyl-1,4-phenylenediamine, 2,2'-dimethylbiphenyl-4,4'-diamine, 2,2'-dimethoxy-4,4'-diaminodiphenyl, 2,2'-diethoxy-4,4'-diaminodiphenyl, 4,4'-diaminodiphenylmethane, 4,4'-diaminodiphenyl ether, 4,4'-diaminodiphenylsulfone, 4,4'-diaminobenzophenone, 1,3-bis(3-aminophenoxy)benzene, 1,3-bis(4-aminophenoxy)benzene, 1,4-bis(4-aminophenoxy)benzene, 4,4'-bis(4-aminophenoxy)biphenyl, bis(4-(3-aminophenoxy)phenyl)sulfone, bis(4-(4-aminophenoxy)phenyl)sulfone, 2,2-bis(4-(4-aminophenoxy)phenyl)propane, 2,2-bis(4-(4-aminophenoxy)phenyl)hexafluoropropane, 2,2-bis(4-(3-aminophenoxy)phenyl)propane, 2,2-bis(4-(3-aminophenoxy)phenyl)hexafluoropropane, 2,2-bis(4-(4-aminophenyl)hexafluoropropane, 2,2-bis(4-aminophenyl)hexafluoropropane, 2,2-bis(3-aminophenyl)hexafluoropropane, 2,2-bis(3-amino-4-hydroxyphenyl)hexafluoropropane, 2,2-bis(3-amino-4-methylphenyl)hexafluoropropane, 4,4'-diaminobenzanilide, and salts thereof (for example, hydrochlorides, sulfates, and nitrates). However, from the viewpoint of obtaining sufficient performance, in all diamine compounds used for the polymer production, preferably 50 to 100 mol%, more preferably 75 to 100 mol%, and still more preferably 90 to 100 mol% is the diamine compound represented by General Formula [DA] or the salt thereof. All diamine compounds used for polymer production may be the diamine compound represented by General formula [DA] or the salt thereof.

In the production of the polyamide, (i) only one diamine compound or a salt thereof corresponding to General Formula [DA] may be used; (ii) diamine compounds or salts thereof corresponding to General Formula [DA] may be used in combination of two or more; or (iii) one or two or more diamine compounds or salts thereof corresponding to General Formula [DA] and one or two or more diamine compounds or salts thereof not corresponding to General Formula [DA] may be used in combination.

Preferred examples of "another compound (monomer) " to be reacted with the diamine compound (monomer) or the salt thereof include dicarboxylic acid or a derivative thereof (diester, dicarboxylic acid halide, active ester compound, and the like). Preferred examples of the dicarboxylic acid or a derivative thereof include a compound represented by General Formula [DC-1] or [DC-2].

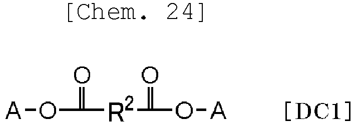

In General Formula [DC1], R² is the same as R² in General Formula [1A] (that is, also the same as R² in General Formula [1]), and two A's each independently represent a hydrogen atom, an alkyl group having 1 to 10 carbon atoms, or an aromatic hydrocarbon group having 6 to 10 carbon atoms.

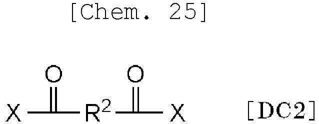

In General Formula [DC2], R² is the same as R² in General Formula [1A] (that is, also the same as R² in General Formula [1]), and two X's each independently represent a fluorine atom, a chlorine atom, a bromine atom, an iodine atom, or an active ester group.

A compound in which X is an "active ester group" is obtained, for example, by reacting a dicarboxylic acid and an active esterification agent in the presence of a dehydration condensation agent. Specific examples of a preferred dehydration condensation agent include dicyclohexylcarbodiimide, 1-ethoxycarbonyl-2-ethoxy-1,2-dihydroquinoline, 1,1'-carbonyldioxy-di-1,2,3-benzotriazole, and N,N'-disuccinimidyl carbonate. Examples of a preferred active esterification agent include N-hydroxysuccinimide, 1-hydroxybenzotriazole, N-hydroxy-5-norbornene-2,3-dicarboxylic imide, ethyl 2-hydroxyimino-2-cyanoacetate, and 2-hydroxyimino-2-cyanoacetic acid amide.

Specific examples of the dicarboxylic acid itself or a dicarboxylic acid from which the dicarboxylic acid derivative is aliphatic dicarboxylic acids such as derivedoxalic acid, malonic acid, succinic acid, glutaric acid, adipic acid, pimelic acid, suberic acid, azelaic acid, sebacic acid; aromatic dicarboxylic acids such as phthalic acid, isophthalic acid, terephthalic acid, 3,3'-dicarboxylic diphenyl ether, 3,4-dicarboxylic diphenyl ether, 4,4'-dicarboxylic diphenyl ether, 3,3'-dicarboxylic diphenylmethane, 3,4-dicarboxylic diphenylmethane, 4,4'-dicarboxyldiphenylmethane, 3,3'-dicarboxyldiphenyldifluoromethane, 3,4-dicarboxyldiphenyldifluoromethane, 4,4'-dicarboxyldiphenyldifluoromethane, 3,3'-dicarboxyldiphenylsulfone, 3,4-dicarboxyldiphenylsulfone, 4,4'-dicarboxyldiphenylsulfone, 3,3'-dicarboxyldiphenylsulfide, 3,4-dicarboxyldiphenylsulfide, 4,4'-dicarboxylic diphenyl sulfide, 3,3'-dicarboxylic diphenyl ketone, 3,4-dicarboxylic diphenyl ketone, 4,4'-dicarboxylic diphenyl ketone, 3,3'-dicarboxylic diphenyl methane, 3,4-dicarboxyldiphenylmethane, 4,4'-dicarboxyldiphenylmethane, 2,2-bis(3-carboxyphenyl)propane, 2,2-bis(3,4'-carboxyphenyl)propane, 2,2-bis(4-carboxyphenyl)propane, 2,2-bis(3-carboxyphenyl)hexafluoropropane, 2,2-bis(3,4'-carboxyphenyl)hexafluoropropane, 2,2-bis(4-carboxyphenyl)hexafluoropropane, 1, 3-bis(3-carboxyphenoxy)benzene, 1,4-bis(3-carboxyphenoxy)benzene, 1,4-bis(4-carboxyphenoxy)benzene, 3,3'-(1,4-phenylenebis(1-methylethylidene))bisbenzoic acid, 3,4'-(1,4-phenylenebis(1-methylethylidene))bisbenzoic acid, 4,4'-(1,4-phenylenebis(1-methylethylidene))bisbenzoic acid, 2,2-bis(4-(3-carboxyphenoxy)phenyl)propane, 2,2-bis(4-(4-carboxyphenoxy)phenyl)propane, 2,2-bis(4-(3-carboxyphenoxy) phenyl )hexafluoropropane, 2,2-bis(4-(4-carboxyphenoxy)phenyl)hexafluoropropane, 2,2-bis(4-(3-carboxyphenoxy)phenyl)sulfide, 2,2-bis(4-(4-carboxyphenoxy)phenyl)sulfide, 2,2-bis(4-(3-carboxyphenoxy)phenyl)sulfone, and 2,2-bis(4-(4-carboxyphenoxy)phenyl)sulfone; perfluorononenyloxy group-containing dicarboxylic acids such as 4-(perfluorononenyloxy)phthalic acid, 5-(perfluorononenyloxy)isophthalic acid, 2-(perfluorononenyloxy)terephthalic acid, and 4-methoxy-5-(perfluorononenyloxy)isophthalic acid; and perfluorohexenyloxy group-containing dicarboxylic acids such as 4-(perfluorohexenyloxy)phthalic acid, 5-(perfluorohexenyloxy)isophthalic acid, 2-(perfluorohexenyloxy)terephthalic acid, and 4-methoxy-5-(perfluorohexenyloxy) isophthalic acid.

The dicarboxylic acid or the derivative thereof may be used alone, or two or more thereof may be used in combination.

Preferred examples of the dicarboxylic acid or the derivative of the dicarboxylic acid include aromatic dicarboxylic acids and derivatives thereof. Particularly preferred examples of the dicarboxylic acid or the derivative of the dicarboxylic acid include the following. In the following, definition and specific aspects of A are the same as those of General Formula [DC-1], and definition and specific aspects of X are the same as those of General Formula [DC-2] .

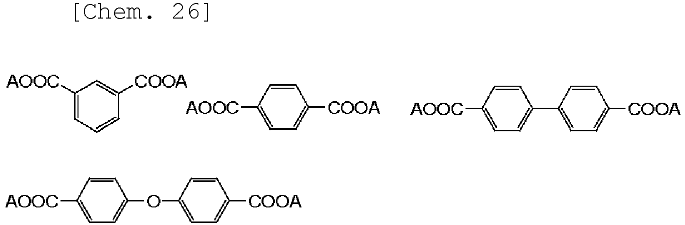

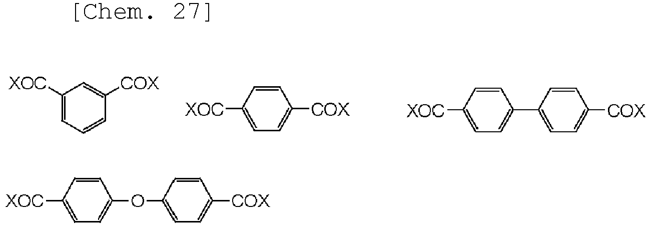

As the organic solvent used for the reaction (polycondensation), a solvent in which the raw material compounds are dissolved can be used without particular limitation. Specific examples thereof include an amide-based solvent, an ether-based solvent, an aromatic solvent, a halogen-based solvent, and a lactone-based solvent. More specific examples thereof include:, as the amide-based solvent, N,N-dimethylformamide, N,N-dimethylacetamide, N-methylformamide, hexamethylphosphoric triamide, or N-methyl-2-pyrrolidone; as the ether-based solvent, diethyl ether, dipropyl ether, diisopropyl ether, dibutyl ether, cyclopentyl methyl ether, diphenyl ether, dimethoxyethane, diethoxyethane, tetrahydrofuran, dioxane, or trioxane; as the aromatic solvent, benzene, anisole, nitrobenzene, or benzonitrile; as the halogen-based solvent, chloroform, dichloromethane, 1,2-dichloroethane, or 1,1,2,2-tetrachloroethane; and as the lactone-based solvent, γ-butyrolactone, γ-valerolactone, ε-valerolactone, γ-caprolactone, ε-caprolactone, or α-methyl-γ-butyrolactone. Only one type of organic solvent may be used, or two or more types thereof may be mixed and used.

The temperature during the reaction may be appropriately set, for example, between -100°C to 100°C. In addition, the reaction may be carried out in an inert gas environment such as nitrogen and argon.

In the production of polyamide, terminal modification with an addition-reactive group, which is often carried out in known polyamide resins, may be carried out.

The addition-reactive group is not particularly limited as long as it is a group which undergoes an addition polymerization reaction (curing reaction) by heating. Any reactive group selected from the group consisting of a group including an acetylene bond, such as a phenylethynyl group, a nadic acid group, and a maleimide group is preferable, a group including an acetylene bond, such as a phenylethynyl group, is more preferable, and a phenylethynyl group is still more preferable.

The addition-reactive group is introduced into the terminal of polymer by reacting a compound having the addition-reactive group and an acid anhydride group or an amino group in one molecule with an amino group or an acid anhydride group of the terminal of polymer. This reaction is preferably a reaction forming an imide ring. Examples of the compound having an acid anhydride group or an amino group together with the addition-reactive group in the molecule include 4-(2-phenylethynyl) phthalic anhydride, phenylethynyl trimellitic anhydride, 4-(2-phenylethynyl) aniline, 4-ethynyl-phthalic anhydride, 4-ethynylaniline, nadic acid anhydride, and maleic acid anhydride.

In addition, in the production of polyamide, the terminal may be capped with dicarboxylic acid anhydrides such as maleic acid anhydride, phthalic acid anhydride, nadic acid anhydride, ethynyl phthalic acid anhydride, and hydroxy phthalic acid anhydride, hydroxyaniline, aminobenzoic acid, dihydroxyaniline, carboxyhydroxyaniline, and dicarboxyaniline.

After completion of the polycondensation reaction, for the purpose of removing residual monomers and low-molecular-weight substances, it is preferable to add the reaction solution to a poor solvent (for example, water, alcohol, and the like) to precipitate, isolate, and purify the polyamide. As a result, polyamides with reduced impurities can be produced, and thus insulating material with better dielectric properties can be obtained.

The polyamide with reduced impurities may be dissolved again in the organic solvent. By doing so, it is possible to obtain a polyamide solution with a small amount of impurities.

### <Method for producing polybenzoxazole>

Using the polyamide produced as described above, a polybenzoxazole can be produced.

Specifically, by a method including
· a first step of producing a polyamide by <Method for producing polyamide> described above, and
· a second step of dehydrating and ring-closing the polyamide obtained in the first step,
the polybenzoxazole according to the present embodiment (polybenzoxazole having the structural unit represented by General Formula [1] described above) can be produced.

The dehydration and ring-closing reaction in the second step is usually carried out by heating. Specifically, the polyamide (polyhydroxyamide) obtained in the first step is heated to 100°C to 350°C. As a result, cyclodehydration proceeds in the polyamide. Then, the polybenzoxazole according to the present embodiment can be obtained.

The heating of the polyamide may be carried out by heating the polyamide solution or by heating the polyamide in a solid form (for example, in a form of a film), preferably the latter. This will be described later in detail as a method for producing an insulating material for a high-frequency electronic component.

### <Method for producing insulating material for high-frequency electronic component and insulating material for high-frequency electronic component>

In the present embodiment, an insulating material for a high-frequency electronic component, which includes the polybenzoxazole, can typically be obtained by heating the above-described polyamide or its solution.

Specifically, the insulating material for a high-frequency electronic component, which includes the polybenzoxazole having the structural unit represented by General Formula [1], can be produced through the following steps. Incidentally, the following drying step and heating step may be performed continuously.
· a step (coating step) of applying the above-described polyamide solution onto a supporting base material
· a step (drying step) of drying the solvent contained in the applied polyamide solution to obtain a resin film including the polyamide
· a step (heating step) of heat-treating the obtained resin film to form a cured film

Each of the above-described steps will be explained below.

### (Coating step)

A coating method in the coating step is not particularly limited, and a known method can be adopted. Depending on coating film thickness, solution viscosity, and the like, a known coating devices such as spin coater, bar coater, doctor blade coater, air knife coater, roll coater, rotary coater, flow coater, die coater, and lip coater can be appropriately used.

The supporting base material is not particularly limited. An inorganic base material or an organic base material is suitable. Specific examples thereof include glass, a silicon wafer, stainless steel, alumina, copper, nickel, polyethylene terephthalate, polyethylene glycol terephthalate, polyethylene glycol naphthalate, polycarbonate, polyimide, polyamide-imide, polyetherimide, polyetheretherketone, polypropylene, polyether sulfone, polyethylene terephthalate, polyphenylene sulfone, and polyphenylene sulfide.

Among these, from the viewpoint of heat resistance, it is preferable to use an inorganic base material, and it is more preferable to use an inorganic base material such as glass, silicon wafer, and stainless steel.

By adjusting the coating amount per unit area in the coating step and the concentration of the polyamide solution used for coating, a thickness of the finally obtained film (cured film and the insulating material for a high-frequency electronic component) can be adjusted.

The thickness of the finally obtained film (cured film and the insulating material for a high-frequency electronic component) is usually 1 um or more and 1000 um or less, preferably 5 um or more and 500 pm or less. In a case where the thickness is 1 um or more, strength of the film itself can be sufficient. In a case where the thickness is 1000 um or less, defects such as cissing, dents, and cracks can be easily suppressed.

### (Drying step)

In the drying step, the solvent in the applied polyamide solution is volatilized by heating using a hot plate. Depending on the type of solvent in which the polyamide is dissolved, the heating temperature in the drying step is preferably 50°C or higher and 250°C or lower, and more preferably 80°C or higher and 200°C or lower. The heating temperature in the drying step is usually lower than a temperature in the subsequent heating step.

In a case where the heating temperature in the drying step is 50°C or higher, the drying can be sufficiently easily performed. In addition, in a case where the heating temperature in the drying step is 250°C or lower, defects such as cissing, dents, and cracks due to rapid solvent evaporation can be suppressed, and a uniform film can be easily formed.

### (Heating step)

In the heating step, the resin film obtained in the drying step is cured by a heat treatment at a high temperature. By heating, the ring-closing reaction of the polyamide in the resin film proceeds, and an insulating material for a high-frequency electronic component (cured film) including the polybenzoxazole can be obtained. In the heating step, it is expected to remove residual solvent which cannot be removed in the drying step, improve cyclization rate, and improve physical properties. The temperature of the heating step is preferably 100°C or higher and 400°C or lower, and more preferably 150°C or higher and 350°C or lower. In a case where the temperature of the heating step is 100°C or higher, the cyclization reaction can be sufficiently progressed easily. In addition, in a case where the temperature of the heating step is 400°C or lower, it is easy to suppress occurrence of defects such as cracks.

The heating step is preferably performed using a device such as an inert gas oven, a hot plate, a box-type dryer, and a conveyor-type dryer, but is not limited to these devices. From the viewpoint of preventing oxidation of the resin film, and removing the residual solvent, the heating step is preferably performed under an inert gas stream. Examples of the inert gas include nitrogen and argon. The oxygen concentration in the inert gas is preferably 500 ppm or less, more preferably 100 ppm or less, and still more preferably 20 ppm or less. By lowering the oxygen concentration, it is easy to suppress coloring and deterioration in performance due to the oxidation of the resin film during heating.

Depending on the application and purpose, a peeling step of peeling off the cured film (containing polybenzoxazole) from the supporting base material after the heating step and using it as a polybenzoxazole substrate may be performed. The peeling step can be performed after cooling from room temperature (20°C) to approximately 40°C after the heating step. A release agent may be applied onto the supporting base material in advance in order to facilitate the peeling. The release agent used in this case is not particularly limited, and examples thereof include silicon-based or fluorine-based release agents.

The insulating material for a high-frequency electronic component (hereinafter, also simply referred to as an "insulating material") according to the present embodiment includes the polybenzoxazole having the structural unit represented by General Formula [1] described above. The insulating material according to the present embodiment may additionally include the polyamide having the structure represented by General Formula [1A] described above.

The insulating material according to the present embodiment is typically film-like. A film-like insulating material can be obtained, for example, by using the polyamide solution and going through the coating step, the drying step, and the heating step, as described above.

For convenience of application to the manufacturing process of electronic devices, it is preferable that the insulating material according to the present embodiment has good heat resistance. A 5%-weight-loss temperature (Td₅) can be used as an index for the heat resistance. As described in Examples later, Td₅ can be quantified by using a differential scanning calorimeter and reading data in a case where the temperature of the insulating material is increased at a constant rate.

Td₅ of the insulating material according to the present embodiment is preferably 400°C or higher, more preferably 410°C or higher, and still more preferably 420°C or higher. Although there is no particular upper limit for Td₅, from the viewpoint of realistic design, the upper limit of Td₅ is, for example, 600°C.

Since the insulating material according to the present embodiment includes the polybenzoxazole having the structural unit represented by General Formula [1], the insulating material according to the present embodiment is preferably used as an insulating material provided in a high-frequency equipment (communication equipment and the like) used in 5G (fifth generation mobile communication system) .

Specifically, a dielectric loss tangent of the insulating material according to the present embodiment at a frequency of 28 GHz is preferably 0.012 or less and more preferably 0.007 or less. In addition, a relative permittivity of the insulating material according to the present embodiment at a frequency of 28 GHz is preferably 3.2 or less and more preferably 3.0 or less. The lower limit value of the relative permittivity is practically 2.0.

By designing the insulating material so that the dielectric loss tangent at a frequency of 28 GHz is 3.2 or less and/or the relative permittivity at a frequency of 28 GHz is 0.012 or less, it is possible to increase transmission speed and sufficiently reduce transmission loss in 5G.

### <High-frequency electronic component and high-frequency equipment>

The high-frequency electronic component according to the present embodiment includes the above-described insulating material. In addition, by using the high-frequency electronic component, a high-frequency equipment (communication terminal and the like) can be produced.

As an example, the high-frequency electronic component according to the present embodiment can be obtained by providing wiring portions on one or both sides of the film-like insulating material. By doing so, the transmission speed can be increased and/or the transmission loss can be reduced.

In addition, the above-described insulating material may have good heat resistance. Therefore, even in a case where the temperature of the insulating material is likely to rise (for example, drying, vapor deposition, plasma treatment, and the like) in the process of producing the high-frequency electronic component, the performance of the insulating material is unlikely to change. This is preferable for the production of electronic parts.

Examples of a method for forming a wiring portion on the film-like insulating material include a method in which the wiring portion is formed by forming a conductive layer consisting of a conductive material such as copper, indium tin oxide (ITO), polythiophene, polyaniline, and polypyrrole by a lamination method, a metallizing method, a sputtering method, a vapor deposition method, a coating method, or a printing method, and then patterning the conductive layer. Before forming the conductive layer, in order to improve adhesion force between the insulating material and the conductive layer, a surface of the film-like insulating material may be modified by a plasma treatment or the like. In addition, an adhesive may be used to improve the adhesion force.

### <Diamine or salt thereof and production method thereof>

A diamine having one trifluoromethyl group represented by General Formula [DA] or a salt thereof, which can be used as a monomer for synthesizing the polybenzoxazole, can be obtained, for example, by reduction (specifically hydrogenation, reduction using hydrazines, and the like) of a bisnitrophenol represented by General Formula [DN] as shown in the reaction formula below. In addition, for example, the salt of the diamine can be obtained by reacting the diamine represented by General Formula [DA] with an acid. The type of the salt is not particularly limited. Examples thereof include hydrochlorides, sulfates, and nitrates.

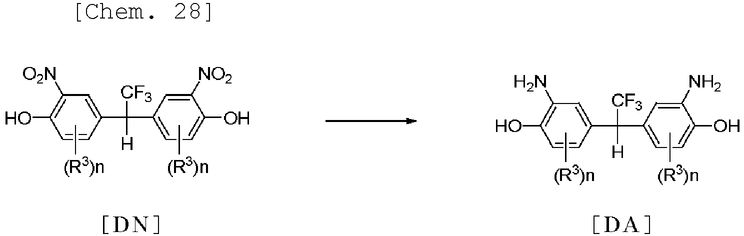

In General Formula [DN], definitions and specific aspects of two n's and R³ are the same as those in General Formula [DA] (that is, the same as n and R³ in General Formula [2]).

The bisnitrophenol represented by General Formula [DN] can be obtained by reacting a mixture of a fluoral and hydrogen fluoride with nitrophenol as shown in the following reaction formula.

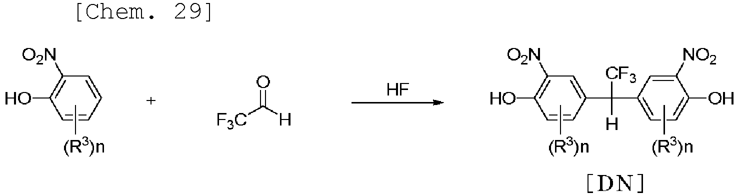

In the above, definitions and specific aspects of two n's and R³ in the general formula of the leftmost nitrophenol are the same as those in General Formula [DN] (that is, the same as n and R³ in General Formula [2]).

For the preparation of fluoral as a starting material, commercially available hydrates (products of Tokyo Chemical Industry Co., Ltd.) and hemiacetal of fluoral can be used as its equivalent. On the other hand, a hydrate of fluoral and a hemiacetal of fluoral can be prepared by methods described in Japanese Unexamined Patent Publication No. H5-97757.

Generally, the fluoral is often used as a hydrate or hemiacetal. Therefore, in a case where the fluoral is used under anhydrous conditions, anhydrous fluoral can be prepared by dehydrating the fluoral hydrate or hemiacetal.

On the other hand, as described in Japanese Unexamined Patent Publication No. H3-184933, catalytic gas-phase fluorination of inexpensive chloral can be converted almost quantitatively to fluoral, which can also be used to prepare anhydrous fluoral (see also Preparation Example 1 later for this).

As described in Japanese Unexamined Patent Publication No. 2019-026628, the fluoral is a low-boiling compound, generally highly self-reactive and difficult to handle, but the fluoral can be handled very stably in a hydrogen fluoride solution and is preferably used. In a case where the fluoral is treated in hydrogen fluoride, 1,2,2,2-tetrafluoroethanol, which is an adduct of fluoral and hydrogen fluoride, is produced as shown in the scheme below (see also Preparation Example 1 later for this).

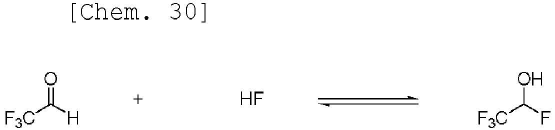

As described above, 1,2,2,2-tetrafluoroethanol forms an equilibrium between fluoral and hydrogen fluoride, and it is considered that the presence of excess hydrogen fluoride in the system maintains the equilibrium state. As a result, it is presumed that the decomposition of fluoral is suppressed. The above-described fluoral in hydrogen fluoride has been confirmed not only to improve the stability of the compound, but also to raise the boiling point, and the fluoral, which is a low-boiling-point compound, can be easily handled as an adduct of hydrogen fluoride at around room temperature.

In a case of treating the prepared fluoral as a mixture with hydrogen fluoride, an amount of hydrogen fluoride added is usually 0.1 to 100 mol with respect to 1 mol of the prepared fluoral, preferably 1 to 75 mol and more preferably 2 to 50 mol. In a case where the amount of hydrogen fluoride added is more than 0.1 mol, a sufficient stabilizing effect is likely to be obtained. In addition, the same stabilizing effect can be expected by adding 100 mol or more of hydrogen fluoride, but it is not preferable in terms of productivity and economy. In addition, the mixture of fluoral and hydrogen fluoride used in this step may contain an excess amount of hydrogen fluoride, but because it has the function as an acidic substance that hydrogen fluoride itself has, it can act effectively as an acid catalyst or a dehydrating agent, and can also be used as an additive to accelerate the reaction in some cases. That is, it can be said that there is an advantage in treating fluoral as the mixture of fluoral and hydrogen fluoride.

The amount of the nitrophenol compound used as a raw material of bisnitrophenol may be 1 mol or more with respect to 1 mol of fluoral. Generally, 2 to 10 mol is preferable because the reaction proceeds smoothly, and 2 to 5 mol is particularly preferable in consideration of post-treatment.

The step of obtaining the bisnitrophenol represented by General Formula [DN] can be performed in the presence of a reaction solvent. Examples of the reaction solvent include an aliphatic hydrocarbon-based solvent, an aromatic hydrocarbon-based solvent, a halogenated hydrocarbon-based solvent, an ether-based solvent, an ester-based solvent, an amide-based solvent, a nitrile-based solvent, and a sulfoxide-based solvent. Specific examples thereof include n-hexane, cyclohexane, n-heptane, benzene, toluene, ethylbenzene, xylene, mesitylene, methylene chloride, chloroform, 1,2-dichloroethane, diethyl ether, tetrahydrofuran, diisopropyl ether, tert-butyl methyl ether, ethyl acetate, n-butyl acetate, N,N-dimethylformamide, N,N-dimethylacetamide, N-methylpyrrolidone, 1,3-dimethyl-2-imidazolidinone, acetonitrile, propionitrile, and dimethylsulfoxide. These reaction solvents can be used alone or in combination.

It is also possible to perform the reaction without using a solvent. As described in Examples later, performing the reaction without a solvent is preferable from the viewpoint that purification operation after the reaction is simple, and a highly pure target product can be obtained only by a simple purification operation.

Since the conversion rate of the condensation reaction can be improved, examples of a preferred aspect of this step include adding Lewis acid or Bronsted acid to the reaction system along with the mixture of fluoral and hydrogen fluoride and the nitrophenol.

Lewis acid which can be used includes metal halides including at least one metal selected from the group consisting of boron (III; oxidation number; hereinafter, the same applies in the present specification), tin (II), tin (IV), titanium (IV), zinc (II), aluminum (III), antimony (III), and antimony (V) . As the metal halide to be used, a metal halide having the maximum possible valence is preferable.

Among metal halides using these metals, boron (III) trifluoride, aluminum (III) trichloride, zinc (II) dichloride, titanium (IV) tetrachloride, tin (IV) tetrachloride, or antimony (V) pentachloride is particularly preferable.

In order for the Lewis acid to function as an additive, the amount to be used is preferably 0.001 mol or more, usually 0.01 to 2.0 mol, per 1 mol of fluoral. However, in a case where the Lewis acid is used in an amount exceeding 2.0 equivalents, it is economically unfavorable.

The Bronsted acid is an inorganic acid or an organic acid, and specific examples of the inorganic acid include phosphoric acid, hydrogen chloride, hydrogen bromide, concentrated nitric acid, concentrated sulfuric acid, fuming nitric acid, and fuming sulfuric acid, and specific examples of the organic acid include formic acid, acetic acid, oxalic acid, benzoic acid, methanesulfonic acid, benzenesulfonic acid, p-toluenesulfonic acid, and trifluoromethanesulfonic acid.

In order for the Bronsted acid to function as an additive, the amount to be used is preferably 0.001 mol or more, usually 0.01 to 2.0 mol, per 1 mol of fluoral. However, in a case where the Bronsted acid is used in an amount exceeding 2.0 equivalents, it is economically unfavorable.

Temperature conditions may be in a range of -20°C to +200°C, usually preferably -10°C to +180°C and particularly preferably 0°C to +160°C.

Pressure conditions may be in a range of atmospheric pressure to 4.0 MPa (absolute pressure; hereinafter, the same applies), usually preferably atmospheric pressure to 2.0 MPa and particularly preferably atmospheric pressure to 1.5 MPa.

Examples of a reaction vessel which can be used include metal containers such as stainless steel, Monel^{™}, Hastelloy^{™}, and nickel; and reaction vessels lined with tetrafluoroethylene resin, chlorotrifluoroethylene resin, vinylidene fluoride resin, PFA resin, propylene resin, polyethylene resin, or the like. It is preferable to use a reactor capable of sufficiently conducting the reaction under normal pressure or increased pressure.

The reaction time is usually within 24 hours. The reaction time may be appropriately adjusted depending on the combination of the mixture of fluoral and hydrogen fluoride and the aryl compound, and the difference in the reaction conditions caused by the amount of Lewis acid and Bronsted acid used as additives. It is preferable to track the progress of the reaction by an analytical unit such as gas chromatography, thin layer chromatography, liquid chromatography, nuclear magnetic resonance, and the like, and to determine the end point of the reaction when the starting material has almost disappeared.

As a post-treatment after the reaction, for example, the reaction-terminated liquid is subjected to a normal purification operation, for example, the reaction-terminated liquid is poured into water or an aqueous solution of an alkali metal inorganic base (for example, sodium hydroxide, potassium hydroxide, sodium hydrogen carbonate, potassium hydrogen carbonate, sodium carbonate, potassium carbonate, and the like) and extracted with an organic solvent (for example, ethyl acetate, toluene, mesitylene, methylene chloride, and the like). By doing so, the desired simple substance of the bisnitrophenol compound represented by General Formula [DN] can be easily obtained. The desired product can be purified to a higher chemical purity product by activated treatment, distillation, recrystallization, column chromatography, and the like, as necessary.

In addition, the step of obtaining the diamine represented by General Formula [DA] can be performed using a general reduction method for reducing a nitro group to an amino group. As a reducing agent, for example, one or two or more of hydrogen, hydrazine, tin, zinc, iron, lithium aluminum hydride, lithium borohydride, sodium aluminum hydride, sodium borohydride, diisobutylaluminum hydride, borane, alane, and the like can be used. Hydrogen or hydrazine is particularly preferable as the reducing agent.

Specific examples of the reduction method include the following.

### (1) Reduction by hydrogenation using metal catalyst

Examples of a reaction solvent which can be used include an aliphatic hydrocarbon-based solvent, an aromatic hydrocarbon-based solvent, a halogenated hydrocarbon-based solvent, an ether-based solvent, an ester-based solvent, an amide-based solvent, and a sulfoxide-based solvent. Specific examples thereof include n-hexane, cyclohexane, n-heptane, benzene, toluene, ethylbenzene, xylene, mesitylene, methylene chloride, chloroform, 1,2-dichloroethane, diethyl ether, tetrahydrofuran, diisopropyl ether, tert-butyl methyl ether, 1,2-methoxyethane, diglyme, ethyl acetate, n-butyl acetate, N,N-dimethylformamide, N,N-dimethylacetamide, N-methylpyrrolidone, 1,3-dimethyl-2-imidazolidinone, and dimethylsulfoxide. The reaction solvent can be used alone or in combination. The amount used is 10 to 2000 parts by mass, more preferably 100 to 1000 parts by mass with respect to 100 parts by mass of the bisnitrophenol compound.

The metal catalyst is, for example, at least one metal (transition metal) selected from the group consisting of ruthenium, palladium, rhodium, and platinum, or a metal compound-supported catalyst in which a metal compound containing the metal is supported on a carrier. Specifically, ruthenium (0 valence), palladium metal (0 valence), rhodiummetal (0 valence), or platinum metal (0 valence); or a compound-supported catalyst in which a ruthenium compound, a palladium compound, a rhodium compound, or a platinum compounds is supported on a carrier can be used.

Preferred examples of the carrier include activated carbon and metal oxide. Specific examples thereof include activated carbon, alumina, zirconia, silica, zeolite, magnesium oxide, and titanium oxide. Examples of the metal compound in the supported catalyst include various compounds such as transition metal acetates, sulfates, nitrates, chlorides, bromides, oxides, and hydroxides.

The transition metal compound-supported catalyst can be prepared by a conventionally known method such as an impregnation method, or a conventionally available product thereof may be used. Among transition metal compound-supported catalysts, in terms of availability, economy, reactivity, and selectivity, a catalyst supported on carbon or alumina is preferable. For example, 5% palladium carbon STD type, 5% rhodium alumina powder, or 2% platinum carbon powder (hydrous product) of N.E. CHEMCAT CORPORATION can be suitably used.

The amount of the transition metal catalyst used is usually, as a metal amount, 0.0001 to 10.00 parts by mass with respect to 100 parts by mass of the bisnitrophenol compound, preferably 0.001 to 5.00 parts by mass and more preferably 0.01 to 2.50 parts by mass. Even in a case where the amount exceeds 10.00 parts by mass, the reactivity is not affected, but there is little merit from the viewpoint of productivity and economy.

Temperature conditions are generally in a range of -20°C to +200°C, preferably -10°C to +180°C and more preferably +20°C to +90°C.

Hydrogen pressure is usually in a range of 0.2 to 4.0 MPa (absolute pressure; hereinafter, the same applies), preferably 0.2 to 2.0 MPa and more preferably 0.4 to 1.0 MPa. Examples of a reaction vessel include metal containers such as stainless steel, Monel^{™}, Hastelloy^{™}, and nickel; and reaction vessels lined with tetrafluoroethylene resin, chlorotrifluoroethylene resin, vinylidene fluoride resin, PFA resin, propylene resin, polyethylene resin, or the like. It is preferable to use a reactor capable of sufficiently conducting the reaction under normal pressure or increased pressure.

The reaction time is usually within 24 hours. The suitable reaction time varies depending on the amount of bisnitrophenol used, the amount of reaction solvent used, the amount of metal catalyst used, and the difference in reaction conditions caused by hydrogen pressure. It is preferable to track the progress of the reaction by an analytical unit such as thin layer chromatography, liquid chromatography, nuclear magnetic resonance, and the like, and to determine the end point of the reaction when the starting substance has almost disappeared.

Post-treatment after the reaction can be carried out, for example, by filtering the reaction-terminated solution to remove the metal catalyst, and then distilling off the solvent to easily obtain the intended simple substance of diamine represented by General Formula [DA]. The desired product can be purified to a higher chemical purity product by activated treatment, distillation, recrystallization, column chromatography, and the like, as necessary.

### (2) Reduction using hydrazines

Examples of the hydrazines which can be used include hydrazine, hydrazine monohydrate, methylhydrazine, hydrazine salt, hydrazine hydrochloride, and hydrazine sulfate. These reducing agents can also be used as solutions such as aqueous solutions, alcohol solutions, and ether solutions.

The amount of hydrazines used is 1.0 to 20.0 mol, more preferably 2.0 to 5.0 mol per 1 mol of the bisnitrophenol.

Examples of a reaction solvent which can be used include a water-based solvent, an alcohol-based solvent, a nitrile-based solvent, an ester-based solvent, an amide-based solvent, and a sulfoxide-based solvent. Specific examples thereof include water, methanol, ethanol, 1-propanol, 2-propanol, tetrahydrofuran, 1,4-dioxane, 1,2-dimethoxyethane, diglyme, ethyl acetate, n-butyl acetate, N,N-dimethylformamide, N,N-dimethylacetamide, N-methylpyrrolidone, 1,3-dimethyl-2-imidazolidinone, and dimethylsulfoxide. These reaction solvents can be used alone or in combination. The amount used is 50 to 2000 parts by mass, more preferably 100 to 1000 parts by mass with respect to 100 parts by mass of the bisnitrophenol.

Examples of the catalyst which can be used include iron halides, Raney nickel, palladium carbon, and ruthenium carbon, and among these, iron halides are particularly preferable. Examples of the iron halide catalyst include hydrates and anhydrides of ferrous chloride, ferric chloride, ferrous bromide, and ferric bromide. The amount of iron halide used is 0.01 to 100 parts by mass, preferably 0.10 to 20.0 parts by mass with respect to 100 parts by mass of the bisnitrophenol.

Temperature conditions are generally -20°C to +200°C, preferably -10°C to +150°C and more preferably +20°C to +100°C.

The reaction time is usually within 24 hours. The reaction time varies depending on the amount of bisnitrophenol used, the amount of reaction solvent used, the amount of metal catalyst used, and the difference in reaction conditions caused by the amount of hydrazines. It is preferable to track the progress of the reaction by an analytical unit such as thin layer chromatography, liquid chromatography, nuclear magnetic resonance, and the like, and to determine the end point of the reaction when the starting material has almost disappeared.

Post-treatment after the reaction can be carried out by simply distilling off the solvent to easily obtain the intended simple substance of diamine represented by General Formula [DA]. The desired product can be purified to a higher chemical purity product by activated treatment, distillation, recrystallization, column chromatography, and the like, as necessary. After the reaction, in a case where it is in a homogeneous state, it can be purified by adding a poor solvent to the solution for reprecipitation.

In the synthesis of diamines involving hydrogenation and nitro group reduction with hydrazine, usually, dehalogenation of the trifluoromethyl group cannot be avoided as a side reaction. Usually, a fluoride ion concentration contained in the diamine after the reduction reaction is 50 ppm or more. In a case where it is subjected to polymerization as it is, it often causes adverse effects on quality such as coloring. Therefore, it is preferable to carry out a treatment to reduce the fluoride ion concentration contained in the monomer raw material. The concentration of fluoride ions contained in the diamine is preferably less than 20 ppm, more preferably less than 10 ppm, and still more preferably less than 2 ppm. The lower the fluorine ion concentration, the better, but in reality, it is, for example, 0.1 ppm or more.

With the production method according to the present embodiment, the diamine having one trifluoromethyl group represented by General Formula [DA] or the salt thereof can be easily produced.

From the cost of raw materials and ease of synthesis, examples of the diamine represented by General Formula [DA] or the salt thereof include a diamine represented by General Formula [DA-2] or [DA-3] or a salt thereof.

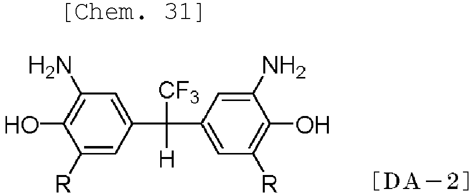

In General Formula [DA-2], two R's each independently represent an alkyl group having 1 to 6 carbon atoms or a halogen atom.

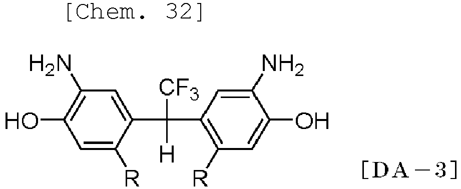

In General Formula [DA-3], two R's each independently represent an alkyl group having 1 to 6 carbon atoms or a halogen atom.

In particular, among the compounds represented by General Formula [DA], compounds represented by the following formulas [DA-4] to [DA-7] or salts thereof can be produced with high purity and high yield by the method described above.

The compound name of the diamine represented by Formula [DA-4] is 1,1,1-trifluoro-2,2-bis(3-amino-5-methyl-4-hydroxyphenyl)ethane.

The compound name of the diamine represented by Formula [DA-5] is 1,1,1-trifluoro-2,2-bis(3-amino-5-fluoro-4-hydroxyphenyl)ethane.

The compound name of the diamine represented by Formula [DA-6] is 1,1,1-trifluoro-2,2-bis(3-amino-5-isopropyl-4-hydroxyphenyl)etha ne.

The compound name of the diamine represented by Formula [DA-7] is 1,1,1-trifluoro-2,2-bis(3-amino-6-methyl-4-hydroxyphenyl)ethane.

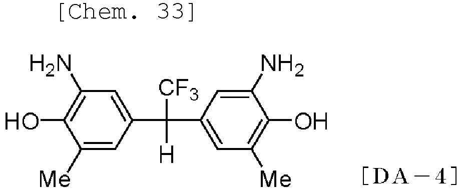

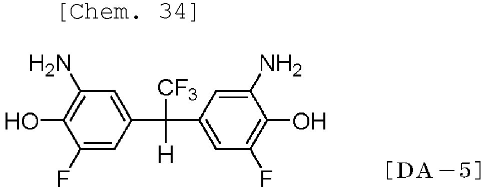

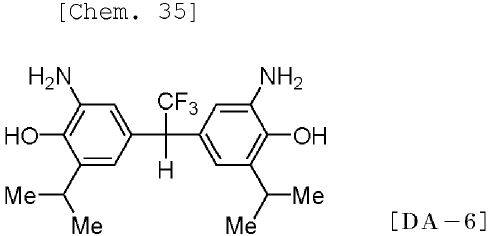

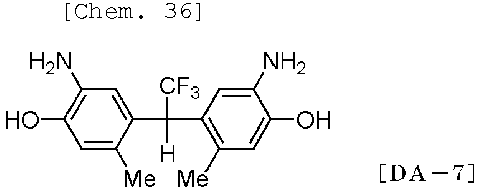

Examples of the diamine represented by General Formula [DA] or the salt thereof also include a diamine represented by Formula [DA-1] or a salt thereof. However, in some cases, the diamine represented by Formula [DA-1] may be excluded from the present embodiment.

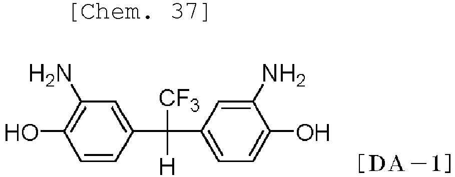

The embodiments of the present invention have been described above, but these are examples of the present invention and various configurations other than the above can be adopted. In addition, the present invention is not limited to the above-described embodiments, and modifications, improvements, and the like within the range in which the object of the present invention can be achieved are included in the present invention.

### [Examples]

Embodiments of the present invention will be described in detail based on Examples and Comparative Examples. It should be noted that the present invention is not limited to Examples only.

### <Various measurements and evaluation methods>

First, various measurements and evaluation methods will be explained.

### [Fluoride ion concentration of diamine]

A solution was prepared by dissolving 1 g of a sample to be measured for fluoride ion concentration in 20 g of ethyl acetate. After adding 10 g of ultrapure water to this solution and shaking well with a separatory funnel, the aqueous layer was separated. To this aqueous layer, 10 mL of TISAB-II (manufactured by ThermoFisher Scientific) was added as an ionic strength adjustment liquid, and the fluoride ion concentration was measured using an ionic electrode (manufactured by ThermoFisher Scientific, ORION VERSASTAR) .

### [Composition analysis]

"%" of a composition analysis value represents the "area%" of a composition, which is obtained by measuring the raw material or product by gas chromatography (hereinafter, referred to as GC; unless otherwise specified, the detector is FID) or liquid chromatography (hereafter, referred to as LC; unless otherwise specified, the detector is UV).

### [Weight-average molecular weight (Mw) and number-average molecular weight (Mn)]

A weight-average molecular weight and a number-average molecular weight were measured using gel permeation chromatography (GPC, HLC-8320 manufactured by TOSOH CORPORATION) with polystyrene as a standard substance. N,N-dimethylformamide (DMF) was used as the mobile phase, and TSKgel SuperHZM-H was used as the column.

### [Infrared absorption spectrum (IR) measurement]

The infrared absorption spectrum of a compound or a film was measured using Nicolet NEXUS470FT-IR (manufactured by ThermoFisher Scientific).

### [Heat resistance]

For the 5%-weight-loss temperature (Td₅), with a simultaneous differential thermal thermogravimetric measurement device (manufactured by Hitachi High-Tech Science Corporation, model name: STA7200), the measurement was performed under the conditions of a temperature increase rate of 10 °C/min from the initial temperature of 30°C.

### [Dielectric properties] (relative permittivity (εr) and dielectric loss tangent (tan δ))

Relative permittivity (εr) and dielectric loss tangent (tan δ) of cured films obtained in Examples below or films of Comparative Examples at a frequency condition of 28 GHz, a temperature of 23°C, and a relative humidity of 50 %RH were measured by a split cylinder resonator method. As a measurement device, a network analyzer device name "N5290A" manufactured by Keysight Technologies and a split cylinder resonator (28 GHz CR-728) manufactured by KANTO Electronic Application and Development Inc. were used.

### [Curing temperature]

For the curing temperature, using a differential scanning calorimeter (manufactured by SII NanoTechnology Inc., model name: X-DSC7000), measurement was performed under the conditions of a starting temperature of 30°C, a measurement temperature range of -40°C to 350°C, and a temperature increase rate of 10 °C/min. Specifically, the temperature at which the maximum value of the endothermic peak generated in a case where the polyamide is ring-closed and converted to the polybenzoxazole was taken as the curing temperature.

### <Synthesis Example of monomer (diamine)>

### [Catalyst Preparation Example]

896 g of special grade reagent CrCl₃·6H₂O was dissolved in pure water to prepare 3.0 L of a solution. 400 g of granular alumina was immersed in this solution and left for a whole day and night. After standing, the solution was filtered to take out the alumina, kept at 100°C in a hot air circulating dryer, and dried for a whole day and night. Chromium-supported alumina was thus obtained.

The obtained chromium-supported alumina was filled in a cylindrical SUS316L reaction tube with a diameter of 4.2 cm and a length of 60 cm, equipped with an electric furnace. The temperature was raised to 300°C while flowing nitrogen gas into the reaction tube at a flow rate of approximately 20 mL/min. In a case where outflow of water was no longer observed, hydrogen fluoride was entrained in the nitrogen gas and its concentration was gradually increased. In a case where a hot spot due to fluorination of the packed chromium-supported alumina reached the outlet end of the reaction tube, the reactor temperature was raised to 350°C and held there for 5 hours. As a result, a catalyst was prepared.

### [Preparation Example 1: Preparation of fluoral represented by following scheme]

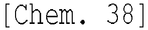

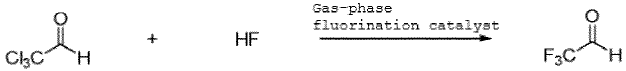

A gas phase reactor (made of SUS316L, diameter: 2.5 cm, length: 40 cm) consisting of a cylindrical reaction tube equipped with an electric furnace was filled with 125 mL of the catalyst prepared in the above-described catalyst preparation example.

The temperature of the reaction tube was raised to 280°C while flowing air at a flow rate of approximately 100 mL/min into the reaction tube filled with the catalyst, and hydrogen fluoride was introduced at a rate of approximately 0.32 g/min over 1 hour. Next, chloral (trichloroacetaldehyde) as a raw material was started to be supplied to the reaction tube at a rate of approximately 0.38 g/min (contact time: 15 seconds) . One hour after the start of the reaction, the reaction was stabilized, so that gas flowing out of the reactor was collected over 18 hours in a cylinder made of SUS304 with a blowing pipe cooled with a coolant of -15°C.

Hydrogen fluoride content, hydrogen chloride content, and organic matter content were calculated by titration for 484.8 g of the fluoral-containing collected liquid obtained above. The results were 40% by mass of hydrogen fluoride, 11% by mass of hydrogen chloride, and 49% by mass of organic matter, and a recovery rate of organic matter was 88% (based on moles of feed chloral). In addition, in a case where a part of the recovered organic matter was collected in a resin NMR tube, and the degree of fluorination was confirmed by ¹⁹F-NMR, it was confirmed that substantially no low-order fluorinated compounds were detected and fluorination was progressing quantitatively.

Next, 450 g (hydrogen fluoride: 40% by mass, hydrogen chloride: 11% by mass, organic matter: 49% by mass) of a part of the collected mixture containing fluoral was charged into a 500 ml SUS reactor equipped with a cooling pipe through which a coolant of -15°C was passed, a thermometer and a stirrer, and the reactor was heated to 25°C. Hydrogen chloride passing through a top tower of the cooling pipe was removed by being absorbed by water, while hydrogen fluoride was refluxed in the cooling pipe under normal pressure. After 5 hours of reflux, the reflux was sampled and the sampled mixture was titrated. Hydrogen fluoride content, hydrogen chloride content, and organic matter content were calculated by titration to be hydrogen fluoride: 44% by mass, hydrogen chloride: 1% by mass, and organic matter: 55% by mass. In addition, a portion of the mixture was collected in a resin NMR tube and subjected to ¹⁹F-NMR measurement. From an integral ratio calculated from the obtained chart, it was confirmed that fluoral in anhydrous hydrogen fluoride was converted to 1,2,2,2-tetrafluoroethanol which is a composition of fluoral and hydrogen fluoride. On the other hand, in a case where the water used to absorb the hydrogen chloride was subjected to titration, although hydrogen fluoride was partially included due to entrainment of droplets, almost no organic matter was included.

### [NMR data]

### 1,2,2,2-tetrafluoroethanol:

¹⁹F-NMR (400 MHz, CFCl₃) δ (ppm): -85.8 (3F, s), -137.8 (1F, d, J = 54.9 Hz)

### Hydrogen fluoride:

¹⁹F-NMR (400 MHz, CFCl₃) δ (ppm) : -193.4 (1F, s)

### (Synthesis Example 1: Synthesis of bisnitrophenol represented by following scheme)

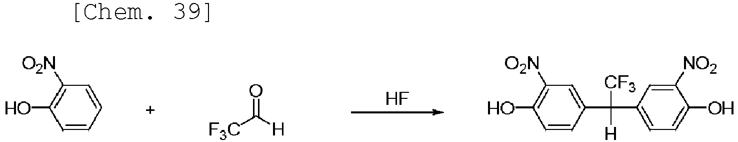

Into a 200 mL stainless steel autoclave reactor equipped with a pressure gauge, a thermometer protection tube, an insert tube, and a stirring motor, 39.2 g (fluoral: 220 mmol, hydrogen fluoride: 862 mmol) of the fluoral-containing mixture obtained in Preparation Example 1 (hydrogen fluoride: 44% by mass, hydrogen chloride: 1% by mass, organic matter: 55% by mass), 74.3 g (3.72 mol) of hydrogen fluoride, and 60.1 g (432 mmol) of 2-nitrophenol were charged, and the mixture was heated in a 120°C oil bath and reacted at an absolute pressure of 1.2 MPa for 18 hours. After the reaction, the reaction solution was poured into a mixture of 200 g of ice water and 270 g of ethyl acetate. The separated organic layer was washed with 240 g of 10% by mass potassium carbonate water and 120 g of water, and then the organic layer was recovered by two-layer separation. After the recovered organic layer was concentrated by an evaporator, it was recrystallized from 67 g of ethyl acetate and 155 g of n-heptane to obtain the target product 1,1,1-trifluoro-2,2-bis(3-nitro-4-hydroxyphenyl)ethane in an amount of 47.0 g, yield: 61%, purity: 99.8% (GC) as a yellow solid.

### [NMR data]

### 1,1,1-trifluoro-2,2-bis(3-nitro-4-hydroxyphenyl)ethane:

¹H-NMR (400 MHz, CDCl₃) δ (ppm) : 4.69 (1H, q, J = 9.4 Hz), 7.20 (2H, d, J = 8.9 Hz), 7.55 (2H, dd, J = 8.7, 2.3 Hz), 8.09 (2H, d, J = 2.3 Hz), 10.57 (2H, s)
¹⁹F-NMR (400 MHz, CDCl₃) δ (ppm): -66.9 (3F, d, J = 8.7 Hz)

### (Synthesis Example 2: Synthesis of diamine represented by following scheme)

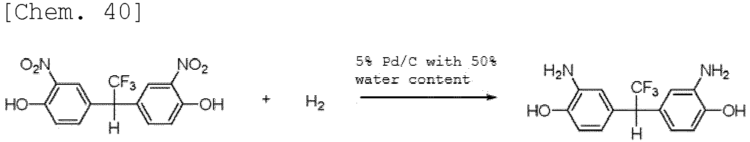

Into a 200 mL stainless steel autoclave reactor equipped with a pressure gauge, a thermometer protection tube, a gas introduction pipe connected to a hydrogen cylinder, and a stirring motor, 22.1 g (61.7 mmol) of 1,1,1-trifluoro-2,2-bis(3-nitro-4-hydroxyphenyl)ethane obtained in Synthesis Example 1, 221 mg of 5% by mass palladium carbon with 50% water content of N.E. CHEMCAT CORPORATION, and 119 g of ethyl acetate were charged, and the mixture was heated in a 70°C oil bath and reacted at an absolute pressure of 0.6 MPa for 9 hours while continuously introducing hydrogen. In a case where a fluoride ion concentration in the reaction solution was measured with an ionic electrode method, it was 80 ppm.

After completion of the reaction, the catalyst was removed by pressure filtration, and then the obtained filtrate was concentrated to be 70 g with an evaporator. By dropping 80 g of toluene into this concentrated liquid to precipitate crystals, the target product 1,1,1,-trifluoro-2,2-bis(3-amino-4-hydroxyphenyl)ethane was obtained in an amount of 15.4 g, yield: 84%, purity: 99.6% (LC) as a white solid. In addition, the fluoride ion concentration of the target product obtained by purification was less than 1 ppm.

### [NMR data]

### 1,1,1-trifluoro-2,2-bis(3-amino-4-hydroxyphenyl)ethane:

¹H-NMR (400 MHz, CD₃CN) δ (ppm) : 4.03 (4H, br-s), 4.45 (1H, q, J = 10.2 Hz), 6.51 (2H, d, J = 8.1 Hz), 6.63 (2H, d, J = 8.7 Hz), 6.67 (2H, s), 6.88 (2H, br-s)
¹⁹F-NMR (400 MHz, CD₃CN) δ (ppm) : -66.1 (3F, d, J = 8.7 Hz)

### (Synthesis Example 3: Synthesis of bisnitrophenol represented by following scheme)

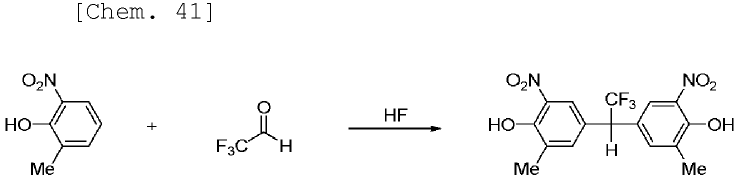

Into a 200 mL stainless steel autoclave reactor equipped with a pressure gauge, a thermometer protection tube, an insert tube, and a stirring motor, 29.4 g (fluoral: 165 mmol, hydrogen fluoride: 647 mmol) of the fluoral-containing mixture obtained in Preparation Example 1 (hydrogen fluoride: 44% by mass, hydrogen chloride: 1% by mass, organic matter: 55% by mass), 49 g (2.45 mol) of hydrogen fluoride, and 50.2 g (328 mmol) of 2-methyl-6-nitrophenol were charged, and the mixture was heated in a 130°C oil bath and reacted at an absolute pressure of 1.4 MPa for 19 hours. After the reaction, the reaction solution was poured into a mixture of 200 g of ice water, 135 g of ethyl acetate, and 135 g of methyl-t-butyl ether. The separated organic layer was washed with 240 g of saturated sodium bicarbonate water and 120 g of water, and then the organic layer was recovered by two-layer separation. After the recovered organic layer was concentrated by an evaporator, the obtained solid was dispersed and washed with 113 g of ethyl acetate and 170 g of n-heptane to obtain the target product 1,1,1-trifluoro-2,2-bis(3-methyl-5-nitro-4-hydroxyphenyl)ethane in an amount of 39.0 g, yield: 62%, purity: 99.8% (GC) as a yellow solid.

### [NMR data]

### 1,1,1-trifluoro-2,2-bis(3-methyl-5-nitro-4-hydroxyphenyl)et hane:

¹H-NMR (400 MHz, CDCl₃) δ (ppm) : 2.33 (6H, s), 4.58 (1H, q, J = 9.3 Hz), 7.37 (2H, d, J = 1.8 Hz), 7.95 (2H, d, J = 1.8 Hz), 10.9 (2H, s)
¹⁹F-NMR (400 MHz, CDCl₃) δ (ppm): -66.9 (3F, d, J = 8.7 Hz)

### (Synthesis Example 4: Synthesis of diamine represented by following scheme)

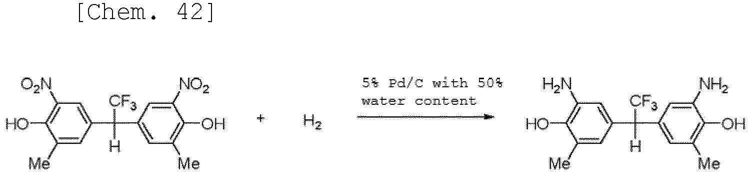

Into a 200 mL stainless steel autoclave reactor equipped with a pressure gauge, a thermometer protection tube, a gas introduction pipe connected to a hydrogen cylinder, and a stirring motor, 28.7 g (74.4 mmol) of 1,1,1-trifluoro-2,2-bis(3-methyl-5-nitro-4-hydroxyphenyl)ethane obtained in Synthesis Example 3, 287 mg of 5% by mass palladium carbon with 50% water content of N.E. CHEMCAT CORPORATION, and 104 g of ethyl acetate were charged, and the mixture was heated in a 70°C oil bath and reacted at an absolute pressure of 0.6 MPa for 17 hours while continuously introducing hydrogen. Analysis of the reaction solution revealed that a selection ratio for 1,1,1-trifluoro-2,2-bis(3-amino-5-methyl-4-hydroxyphenyl)ethane was 98%.

After completion of the reaction, the catalyst was removed by pressure filtration, and then the filtrate was concentrated with an evaporator. As a result, the target product 1,1,1-trifluoro-2,2-bis(3-amino-5-methyl-4-hydroxyphenyl)ethane was obtained in an amount of 28.8 g, yield: 94%, purity: 98.2% (LC) .

### [NMR data]

### 1,1,1-trifluoro-2,2-bis(3-amino-5-methyl-4-hydroxyphenyl)et hane:

¹H-NMR (400 MHz, CD3CN) δ (ppm): 2.12 (6H, s), 2.50 (6H, br-s), 4.34 (1H, q, J = 10.9 Hz), 6.45 (2H, s), 6.57 (2H, s)
¹⁹F-NMR (400 MHz, CD3CN) δ (ppm) : -64.5 (3F, d, J = 8.7 Hz)

### (Synthesis Example 5: Synthesis of bisnitrophenol represented by following scheme)

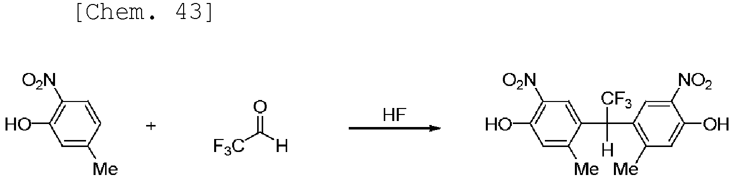

Into a 200 mL stainless steel autoclave reactor equipped with a pressure gauge, a thermometer protection tube, an insert tube, and a stirring motor, 44.7 g (fluoral: 251 mmol, hydrogen fluoride: 983 mmol) of the fluoral-containing mixture obtained in Preparation Example 1 (hydrogen fluoride: 44% by mass, hydrogen chloride: 1% by mass, organic matter: 55% by mass), 59.8 g (2.99 mol) of hydrogen fluoride, and 75.0 g (489 mmol) of 3-methyl-6-nitrophenol were charged, and the mixture was heated in a 120°C oil bath and reacted at an absolute pressure of 1.0 MPa for 17 hours. After the reaction, the reaction solution was poured into a mixture of 225 g of ice water and 405 g of ethyl acetate. The separated organic layer was washed with 300 g of 10% potassium carbonate aqueous solution and 150 g of water, and then the organic layer was recovered by two-layer separation. After the recovered organic layer was concentrated by an evaporator, it was recrystallized from 135 g of ethyl acetate and 205 g of n-heptane to obtain the target product 1,1,1-trifluoro-2,2-bis(2-methyl-5-nitro-4-hydroxyphenyl)ethane in an amount of 46.3 g, yield: 49%, purity: 92.6% (LC) as a brown solid.

### [NMR data]

### 1,1,1-trifluoro-2,2-bis(3-methyl-5-nitro-4-hydroxyphenyl)et hane:

¹H-NMR (400 MHz, CDCl₃) δ (ppm) : 2.33 (6H, s), 4.89 (1H, q, J = 8.7 Hz), 7.03 (2H, d, s), 8.07 (2H, s), 10.5 (2H, s)
¹⁹F-NMR (400 MHz, CDCl₃) δ (ppm) : -65.5 (3F, d, J = 8.7 Hz)

### (Synthesis Example 6: Synthesis of diamine represented by following scheme)

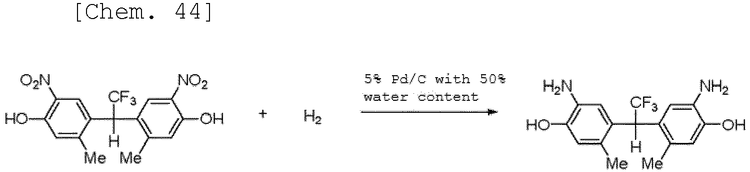

Into a 200 mL stainless steel autoclave reactor equipped with a pressure gauge, a thermometer protection tube, a gas introduction pipe connected to a hydrogen cylinder, and a stirring motor, 19.5 g (50.6 mmol) of 1,1,1-trifluoro-2,2-bis(3-methyl-5-nitro-4-hydroxyphenyl)ethane obtained in Synthesis Example 5, 195 mg of 5% by mass palladium carbon with 50% water content of N.E. CHEMCAT CORPORATION, and 140 g of tetrahydrofuran were charged, and the mixture was heated in a 70°C oil bath and reacted at an absolute pressure of 0.6 MPa for 17 hours while continuously introducing hydrogen. In a case where a fluoride ion concentration in the reaction solution was measured with an ionic electrode method, it was 55 ppm.

After completion of the reaction, the catalyst was removed by pressure filtration, and then the filtrate was concentrated to be 141 g with an evaporator. By dropping 95 g of n-heptane into this concentrated liquid to precipitate crystals, the target product 1,1,1,-trifluoro-2,2-bis(3-amino-6-methyl-4-hydroxyphenyl)ethane was obtained in an amount of 15.3 g, yield: 93%, purity: 99.8% (LC) as a white solid. In addition, the fluoride ion concentration of the target product was less than 1 ppm.

### [NMR data]

### 1,1,1-trifluoro-2,2-bis(3-amino-6-methyl-4-hydroxyphenyl)et hane:

¹H-NMR (400 MHz, CD₃CN) δ (ppm): 2.08 (6H, s), 3.78 (4H, br-s), 4.80 (1H, q, J = 10.1 Hz), 6.49 (2H, s), 6.69 (2H, s), 6.84 (2H, br-s)
¹⁹F-NMR (400 MHz, CD₃CN) δ (ppm) : -63.5 (3F, d, J = 8.7 Hz)

### (Synthesis Example 7: Synthesis of bisnitrophenol represented by following scheme)

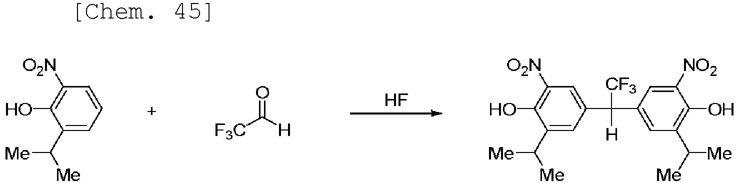

Into a 200 mL stainless steel autoclave reactor equipped with a pressure gauge, a thermometer protection tube, an insert tube, and a stirring motor, 16.9 g (fluoral: 95 mmol, hydrogen fluoride: 372 mmol) of the fluoral-containing mixture obtained in Preparation Example 1 (hydrogen fluoride: 44% by mass, hydrogen chloride: 1% by mass, organic matter: 55% by mass), 29.7 g (1.48 mol) of hydrogen fluoride, and 33.8 g (187 mmol) of 2-isopropyl-6-nitrophenol were charged, and the mixture was heated in a 130°C oil bath and reacted at an absolute pressure of 1.4 MPa for 18 hours . The reaction solution was poured into a mixture of 200 g of ice water and 135 g of ethyl acetate. The separated organic layer was washed with 200 g of saturated sodium bicarbonate water and 100 g of water, and then the organic layer was recovered by two-layer separation. After the recovered organic layer was concentrated by an evaporator, it was recrystallized from 36 g of ethyl acetate and 82 g of n-heptane to obtain the target product 1,1,1-trifluoro-2,2-bis(3-isopropyl-5-nitro-4-hydroxyphenyl)etha ne in an amount of 27.2 g, yield: 66%, purity: 97.2% (LC) as a yellow solid.

### [NMR data]

### 1,1,1-trifluoro-2,2-bis(3-isopropyl-5-nitro-4-hydroxyphenyl )ethane:

¹H-NMR (400 MHz, CDCl₃) δ (ppm) : 1.25 (12H, d, J = 6.9 Hz), 3.42 (2H, sept, J = 6.9 Hz), 4.63 (1H, q, J = 9.4 Hz), 7.47 (2H, d, J = 2.3 Hz), 7.95 (2H, d, J = 2.3 Hz), 11.1 (2H, s)
¹⁹F-NMR (400 MHz, CDCl₃) δ (ppm) : -66.3 (3F, d, J = 8.7 Hz)

### (Synthesis Example 8: Synthesis of diamine represented by following scheme)

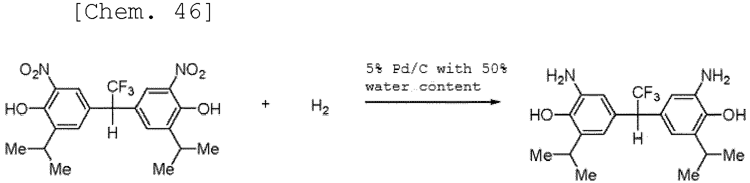

Into a 200 mL stainless steel autoclave reactor equipped with a pressure gauge, a thermometer protection tube, a gas introduction pipe connected to a hydrogen cylinder, and a stirring motor, 14.0 g (31.6 mmol) of 1,1,1-trifluoro-2,2-bis(3-isopropyl-5-nitro-4-hydroxyphenyl)etha ne obtained in Synthesis Example 7, 140 mg of 5% by mass palladium carbon with 50% water content of N.E. CHEMCAT CORPORATION, and 75 g of ethyl acetate were charged, and the mixture was heated in a 70°C oil bath and reacted at an absolute pressure of 0.6 MPa for 16 hours while continuously introducing hydrogen. In a case where a fluoride ion concentration in the reaction solution was measured with an ionic electrode method, it was 70 ppm.

After completion of the reaction, the catalyst was removed by pressure filtration, and then the filtrate was concentrated to be 32 g with an evaporator. By dropping 18 g of n-heptane into this concentrated liquid to precipitate crystals, the target product 1,1,-trifluoro-2,2-bis(3-amino-5-isopropyl-4-hydroxyphenyl)ethan e was obtained in an amount of 11.3 g, yield: 85%, purity: 99.4% (LC) as a white solid. In addition, the fluoride ion concentration of the target product was 1 ppm.

### [NMR data]

### 1,1,1-trifluoro-2,2-bis(3-amino-5-isopropyl-4-hydroxyphenyl )ethane:

¹H-NMR (400 MHz, CD₃OD) δ (ppm) : 1.15 (12H, d, J = 6.9 Hz), 3.22 (2H, sept, J = 7.1 Hz), 4.38 (1H, q, J = 10.3 Hz), 6.62 (2H, s), 6.64 (2H, s)
¹⁹F-NMR (400 MHz, CD₃OD) δ (ppm): -66.6 (3F, d, J = 8.7 Hz)

### (Synthesis Example 9: Synthesis of bisnitrophenol represented by following scheme)

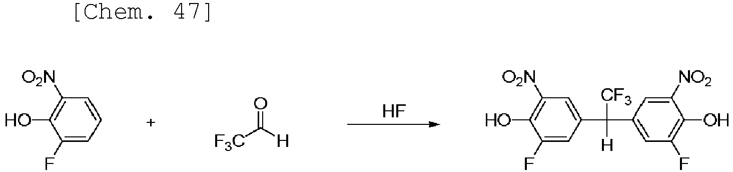

Into a 300 mL stainless steel autoclave reactor equipped with a pressure gauge, a thermometer protection tube, an insert tube, and a stirring motor, 47.8 g (fluoral: 268 mmol, hydrogen fluoride: 1.05 mol) of the fluoral-containing mixture obtained in Preparation Example 1 (hydrogen fluoride: 44% by mass, hydrogen chloride: 1% by mass, organic matter: 55% by mass), 83.7 g (1.48 mol) of hydrogen fluoride, and 82.7 g (527 mmol) of 2-fluoro-6-nitrophenol were charged, and the mixture was heated in a 130°C oil bath and reacted at an absolute pressure of 1.4 MPa for 18 hours. After the reaction, the reaction solution was poured into a mixture of 320 g of ice water and 288 g of ethyl acetate. The obtained organic layer was washed with 500 g of saturated sodium bicarbonate water and 250 g of water, and then the organic layer was recovered by two-layer separation. After the recovered organic layer was concentrated by an evaporator, it was recrystallized from 40 g of ethyl acetate and 104 g of methylcyclohexane to obtain the target product 1,1,1-trifluoro-2,2-bis(3-fluoro-5-nitro-4-hydroxyphenyl)ethane in an amount of 62.9 g, yield: 61%, purity: 95.7% (LC) as a yellow solid.

### [NMR data]

### 1,1,1-trifluoro-2,2-bis(3-fluoro-5-nitro-4-hydroxyphenyl)et hane:

¹H-NMR (400 MHz, CDCl₃) δ (ppm) : 4.67 (1H, q, J = 8.9 Hz), 7.40 (2H, dd, J = 10.3, 1.8 Hz), 7.91 (2H, d, J = 1.8 Hz), 10.5 (2H, s)
¹⁹F-NMR (400MHz, CDCl₃) δ (ppm) : -66.3 (3F, d, J = 8.7 Hz), -128.0 (3F, d, J = 11.6 Hz)

### (Synthesis Example 10: Synthesis of diamine represented by following scheme)

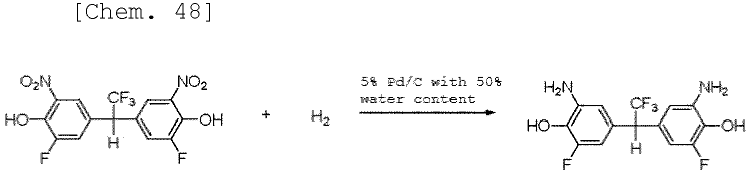

Into a 200 mL stainless steel autoclave reactor equipped with a pressure gauge, a thermometer protection tube, a gas introduction pipe connected to a hydrogen cylinder, and a stirring motor, 29.2 g (74.1 mmol) of 1,1,1-trifluoro-2,2-bis(3-fluoro-S-nitro-4-hydroxyphenyl)ethane obtained in Synthesis Example 9, 292 mg of 5% by mass palladium carbon with 50% water content of N.E. CHEMCAT CORPORATION, and 108 g of ethyl acetate were charged, and the mixture was heated in a 70°C oil bath and reacted at an absolute pressure of 0.6 MPa for 16 hours while continuously introducing hydrogen. In a case where a fluoride ion concentration in the reaction solution was measured with an ionic electrode method, it was 85 ppm.

After completion of the reaction, the catalyst was removed by pressure filtration, and then the obtained filtrate was concentrated with an evaporator to obtain 25 g of solid. The obtained solid was dispersed and washed with 45 g of ethyl acetate and 39 g of methylcyclohexane to obtain the target product 1,1,1-trifluoro-2,2-bis(3-amino-5-fluoro-4-hydroxyphenyl)ethane in an amount of 22.2 g, yield: 90%, purity: 99.7% (LC) as a white solid. In addition, the fluoride ion concentration of the target product was less than 1 ppm.

### [NMR data]

### 1,1,1-trifluoro-2,2-bis(3-amino-5-fluoro-4-hydroxyphenyl)et hane:

¹H-NMR (400 MHz, CD₃CN) δ (ppm) : 1.91 (2H, br-s), 4.22 (2H, br-s), 4.44 (1H, q, J = 10.3 Hz), 6.43 (2H, d, J = 11.4 Hz), 6.49 (2H, s)
¹⁹F-NMR (400 MHz, CD₃CN) δ (ppm): -66.9 (3F, d, J = 10.6 Hz), -138.7 (2F, d, J = 11.6 Hz)

### (Synthesis Example 11: Synthesis of diamine represented by following scheme)

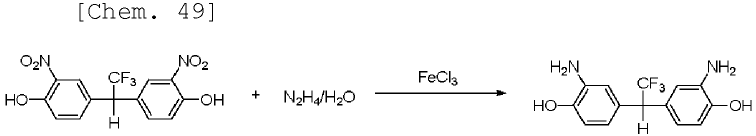

Into a 200 mL four-necked flask equipped with a thermometer, a condenser, an addition funnel, and a stirrer, 10.0 g (28.0 mmol) of 1,1,1-trifluoro-2,2-bis(3-nitro-4-hydroxyphenyl)ethane obtained in Synthesis Example 1, 50 mg of ferric chloride, and 25 g of acetonitrile were charged, and 4.9 g (98.0 mmol) of hydrazine monohydrate was added dropwise thereto over 15 minutes while maintaining the internal temperature at 65°C to 75°C. After the dropwise addition, the reaction was further carried out at an internal temperature of 75°C for 2 hours. In a case where a fluoride ion concentration in the reaction solution was measured with an ionic electrode method, it was 95 ppm.

After completion of the reaction, the reaction solution was cooled to 50°C, and 70 g of water was added dropwise thereto to precipitate crystals, thereby obtaining the target product 1,1,1-trifluoro-2,2-bis(3-amino-4-hydroxyphenyl)ethane in an amount of 7.2 g, yield: 86%, purity: 99.9% (LC) as a white solid. The fluoride ion concentration of the target product was less than 1 ppm.

### <Production of polyamide and polybenzoxazole>

### [Example 1]

Into a 500 mL three-necked flask equipped with a nitrogen inlet tube and a stirrer under ice cooling, 6.0 g (20 mmol) of 2,2-bis(3-amino-4-hydroxyphenyl)-1,1,1-trifluoroethane (BIS-AP-EF), represented by the following formula, and 5.9 g (20 mmol) of 4,4'-oxybis(benzoylchloride) (hereinafter, may be referred to as OBBC) were charged, and 3.6 g (46 mmol) of pyridine as a base and 75 g of N-methylpyrrolidone (NMP) as an organic solvent were added thereto. Under a nitrogen atmosphere, the mixture was stirred at room temperature (25°C) for 5 hours to obtain a reaction solution. Thereafter, the reaction solution was filtered under pressure to prepare a solution of polyhydroxyamide. As a result of GPC measurement of the solution, Mw was 88044 and Mw/Mn was 2.2.

The prepared solution of polyhydroxyamide was applied onto a glass substrate using a spin coater. Thereafter, under a nitrogen atmosphere, heating was performed at a temperature of 110°C for 10 minutes as a drying step, and then heating at 200°C for 30 minutes and at 300°C for 2 hours was performed as a curing step to obtain a film (cured film) on the glass substrate. Thereafter, the film was cooled to room temperature, and peeled off from the glass substrate to obtain a film-like cured film (insulating material). In a case where a film thickness of the cured film was measured, it was 20 um.

From FT-IR measurement results of the cured film, there was absorption specific to oxazole at 1030 to 1100 cm⁻¹. That is, it was confirmed that the cured film contained polybenzoxazole.

For reference, starting materials and reaction schemes are shown below.

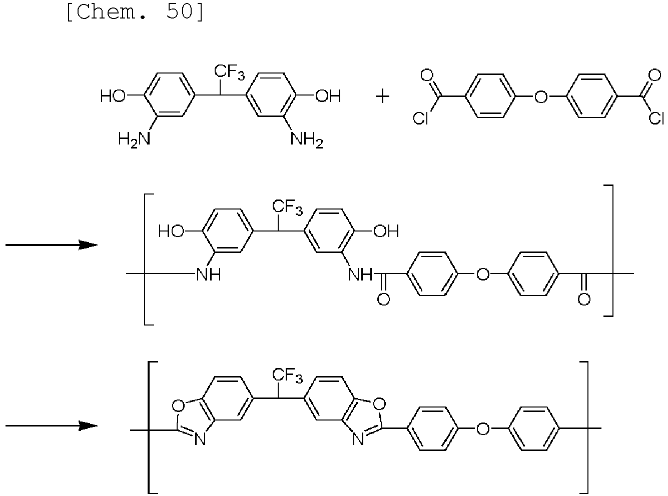

### [Example 2]

Into a 500 mL three-necked flask equipped with a nitrogen inlet tube and a stirrer under ice cooling, 8.0 g (20 mmol) of 2,2-bis(3-amino-4-hydroxyphenyl-5-methyl)-1,1,1-trifluoroethane hydrochloride (BIS-5-MAP-EF•2HCl, hydrochloride of the diamine obtained in Synthesis Example 4), represented by the following formula, and 5.9 g (20 mmol) of OBBC were charged, and 7.9 g (100 mmol) of pyridine as a base and 105 g of NMP as an organic solvent were added thereto. Under a nitrogen atmosphere, the mixture was stirred at room temperature (25°C) for 5 hours to obtain a reaction solution. Thereafter, the reaction solution was filtered under pressure to prepare a solution of polyhydroxyamide. As a result of GPC measurement of the solution, Mw was 97141 and Mw/Mn was 1.7.

The prepared solution of polyhydroxyamide was applied onto a glass substrate using a spin coater. Thereafter, under a nitrogen atmosphere, heating was performed at a temperature of 110°C for 10 minutes as a drying step, and then heating at 200°C for 30 minutes and at 300°C for 2 hours was performed as a curing step to obtain a film (cured film) on the glass substrate. Thereafter, the film was cooled to room temperature, and peeled off from the glass substrate to obtain a film-like cured film (insulating material). In a case where a film thickness of the cured film was measured, it was 20 um.

From FT-IR measurement results of the cured film, there was absorption specific to oxazole at 1030 to 1100 cm⁻¹. That is, it was confirmed that the cured film contained polybenzoxazole.

For reference, starting materials and reaction schemes are shown below.

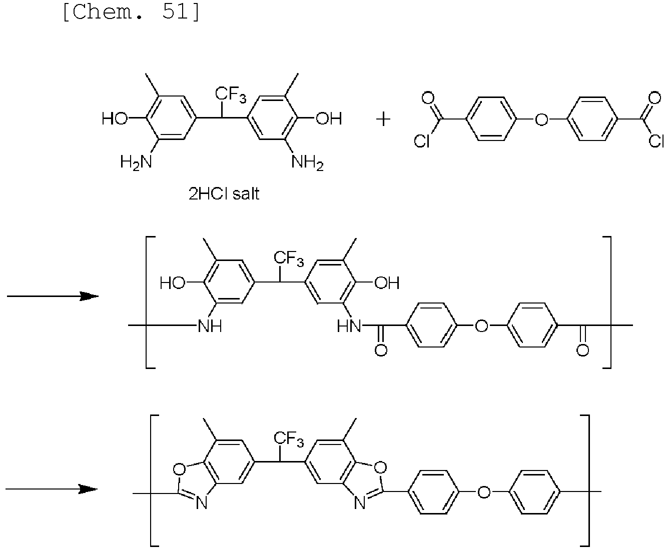

### [Example 3]

Into a 500 mL three-necked flask equipped with a nitrogen inlet tube and a stirrer under ice cooling, 5.4 g (20 mmol) of 2,2-bis(3-amino-4-hydroxyphenyl-6-methyl)-1,1,1-trifluoroethane (BIS-6-MAP-EF), represented by the following formula, and 5.9 g (20 mmol) of OBBC were charged, and 3.6 g (46 mmol) of pyridine as a base and 55 g of NMP as an organic solvent were added thereto. Under a nitrogen atmosphere, the mixture was stirred at room temperature (25°C) for 5 hours to obtain a reaction solution. Thereafter, the reaction solution was filtered under pressure to prepare a solution of polyhydroxyamide. As a result of GPC measurement of the solution, Mw was 63438 and Mw/Mn was 2.2.

The prepared solution of polyhydroxyamide was applied onto a glass substrate using a spin coater. Thereafter, under a nitrogen atmosphere, heating was performed at a temperature of 110°C for 10 minutes as a drying step, and then heating at 200°C for 30 minutes and at 300°C for 2 hours was performed as a curing step to obtain a film (cured film) on the glass substrate. Thereafter, the film was cooled to room temperature, and peeled off from the glass substrate to obtain a film-like cured film (insulating material). In a case where a film thickness of the cured film was measured, it was 14 um.

From FT-IR measurement results of the cured film, there was absorption specific to oxazole at 1030 to 1100 cm⁻¹. That is, it was confirmed that the cured film contained polybenzoxazole.

For reference, starting materials and reaction schemes are shown below.

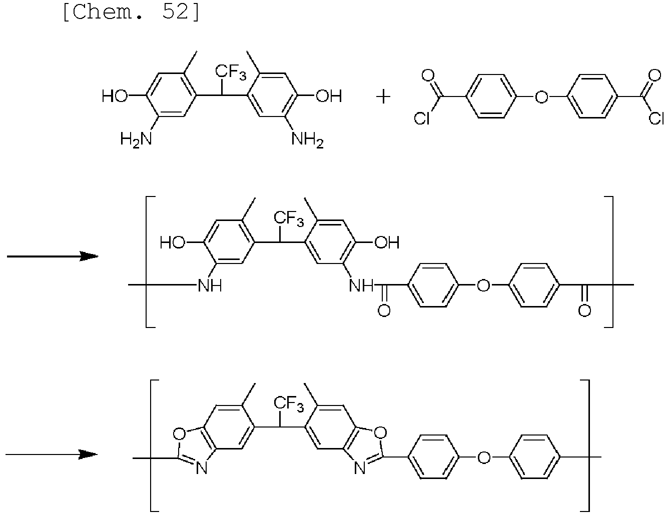

### [Example 4]

Into a 500 mL three-necked flask equipped with a nitrogen inlet tube and a stirrer under ice cooling, 2.8 g (10 mmol) of 2,2-bis(3-amino-4-hydroxyphenyl)-1,1,1-trifluoroethane (BIS-5-FAP-EF), represented by the following formula, 3.7 g (10 mmol) of 2,2-bis(3-amino-4-hydroxyphenyl)-hexafluoropropane (BIS-AP-AF), and 5.9 g (20 mmol) of OBBC were charged, and 3.6 g (46 mmol) of pyridine as a base and 145 g of NMP as an organic solvent were added thereto. Under a nitrogen atmosphere, the mixture was stirred at room temperature (25°C) for 5 hours to obtain a reaction solution. Thereafter, the reaction solution was filtered under pressure to prepare a solution of polyhydroxyamide. As a result of GPC measurement of the solution, Mw was 85147 and Mw/Mn was 1.9.

The prepared solution of polyhydroxyamide was applied onto a glass substrate using a spin coater. Thereafter, under a nitrogen atmosphere, heating was performed at a temperature of 110°C for 10 minutes as a drying step, and then heating at 200°C for 30 minutes and at 300°C for 2 hours was performed as a curing step to obtain a film (cured film) on the glass substrate. Thereafter, the film was cooled to room temperature, and peeled off from the glass substrate to obtain a film-like cured film (insulating material). In a case where a film thickness of the cured film was measured, it was 23 um.

From FT-IR measurement results of the cured film, there was absorption specific to oxazole at 1030 to 1100 cm⁻¹. That is, it was confirmed that the cured film contained polybenzoxazole.

For reference, starting materials and reaction schemes are shown below.

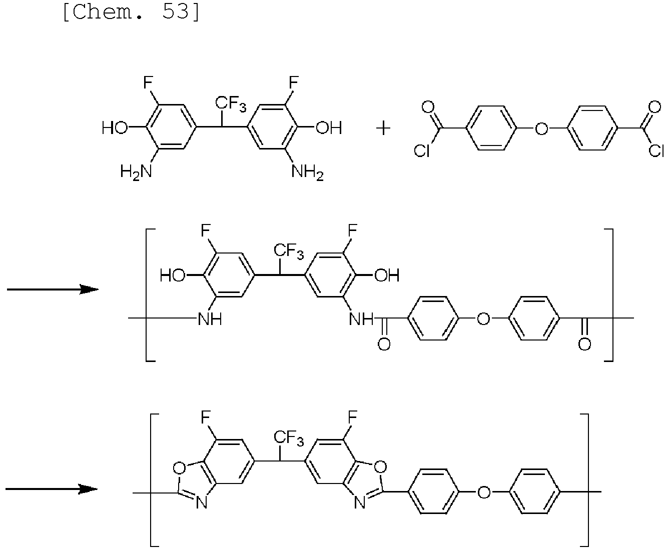

### [Example 5]

Into a 500 mL three-necked flask equipped with a nitrogen inlet tube and a stirrer under ice cooling, 3.8 g (10 mmol) of 2,2-bis(3-amino-4-hydroxyphenyl-5-isopropyl)-1,1,1-trifluoroetha ne (BIS-5-IAP-EF), represented by the following formula, and 3.0 g (10 mmol) of OBBC were charged, and 1.8 g (23 mmol) of pyridine as a base and 33 g of NMP as an organic solvent were added thereto. Under a nitrogen atmosphere, the mixture was stirred at room temperature (25°C) for 5 hours to obtain a reaction solution. Thereafter, the reaction solution was filtered under pressure to prepare a solution of polyhydroxyamide. As a result of GPC measurement of the solution, Mw was 110813 and Mw/Mn was 1.9.

The prepared solution of polyhydroxyamide was applied onto a glass substrate using a spin coater. Thereafter, under a nitrogen atmosphere, heating was performed at a temperature of 110°C for 10 minutes as a drying step, and then heating at 200°C for 30 minutes and at 300°C for 2 hours was performed as a curing step to obtain a film (cured film) on the glass substrate. Thereafter, the film was cooled to room temperature, and peeled off from the glass substrate to obtain a film-like cured film (insulating material). In a case where a film thickness of the cured film was measured, it was 18 um.

From FT-IR measurement results of the cured film, there was absorption specific to oxazole at 1030 to 1100 cm⁻¹. That is, it was confirmed that the cured film contained polybenzoxazole.

For reference, starting materials and reaction schemes are shown below.

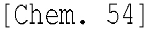

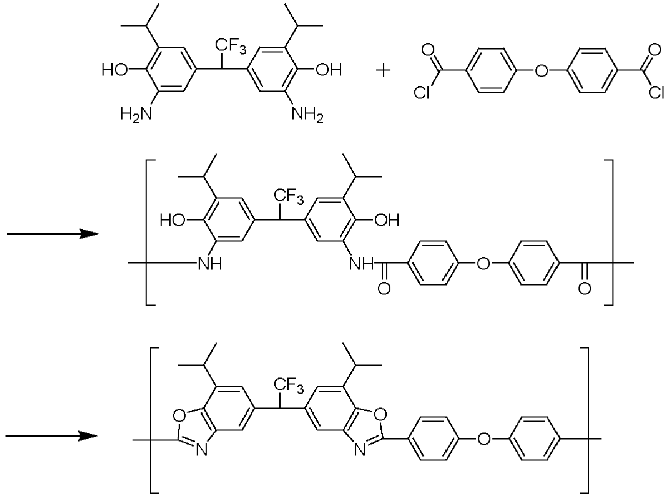

### [Example 6]

Into a 500 mL three-necked flask equipped with a nitrogen inlet tube and a stirrer under ice cooling, 6.0 g (20 mmol) of 2,2-bis(3-amino-4-hydroxyphenyl)-1,1,1-trifluoroethane (BIS-AP-EF), represented by the following formula, and 4.1 g (20 mmol) of isophthaloyl chloride (hereinafter, may be referred to as IPC) were charged, and 3.6 g (46 mmol) of pyridine as a base and 70 g of NMP as an organic solvent were added thereto. Under a nitrogen atmosphere, the mixture was stirred at room temperature (25°C) for 5 hours to obtain a reaction solution. Thereafter, the reaction solution was filtered under pressure to prepare a solution of polyhydroxyamide. As a result of GPC measurement of the solution, Mw was 39967 and Mw/Mn was 1.9.

The prepared solution of polyhydroxyamide was applied onto a glass substrate using a spin coater. Thereafter, under a nitrogen atmosphere, heating was performed at a temperature of 110°C for 10 minutes as a drying step, and then heating at 200°C for 30 minutes and at 300°C for 2 hours was performed as a curing step to obtain a film (cured film) on the glass substrate. Thereafter, the film was cooled to room temperature, and peeled off from the glass substrate to obtain a film-like cured film (insulating material). In a case where a film thickness of the cured film was measured, it was 16 um.

From FT-IR measurement results of the cured film, there was absorption specific to oxazole at 1030 to 1100 cm⁻¹. That is, it was confirmed that the cured film contained polybenzoxazole.

For reference, starting materials and reaction schemes are shown below.

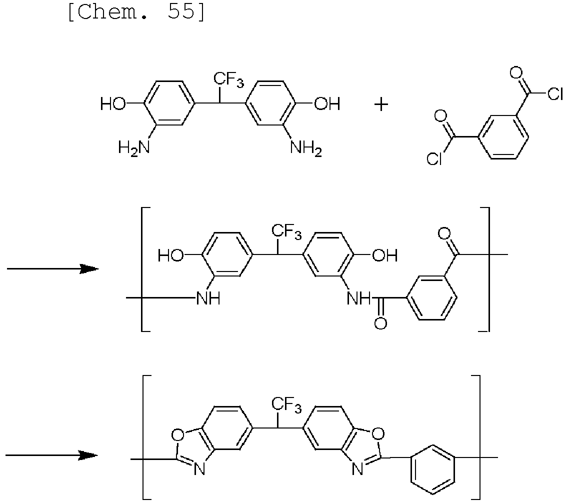

### [Example 7]

Into a 500 mL three-necked flask equipped with a nitrogen inlet tube and a stirrer under ice cooling, 8.0 g (20 mmol) of 2,2-bis(3-amino-4-hydroxyphenyl-5-methyl)-1,1,1-trifluoroethane hydrochloride (BIS-5-MAP-EF•2HCl), represented by the following formula, and 4.1 g (20 mmol) of IPC were charged, and 7.9 g (100 mmol) of pyridine as a base and 100 g of NMP as an organic solvent were added thereto. Under a nitrogen atmosphere, the mixture was stirred at room temperature (25°C) for 5 hours to obtain a reaction solution. Thereafter, the reaction solution was filtered under pressure to prepare a solution of polyhydroxyamide. As a result of GPC measurement of the solution, Mw was 45077 and Mw/Mn was 1.8.

The prepared solution of polyhydroxyamide was applied onto a glass substrate using a spin coater. Thereafter, under a nitrogen atmosphere, heating was performed at a temperature of 110°C for 10 minutes as a drying step, and then heating at 200°C for 30 minutes and at a temperature of 300°C for 2 hours was performed as a curing step to obtain a film (cured film) on the glass substrate. Thereafter, the film was cooled to room temperature, and peeled off from the glass substrate to obtain a film-like cured film (insulating material). In a case where a film thickness of the cured film was measured, it was 21 um.

From FT-IR measurement results, there was absorption specific to oxazole at 1030 to 1100 cm⁻¹. That is, it was confirmed that the cured film contained polybenzoxazole.

For reference, starting materials and reaction schemes are shown below.

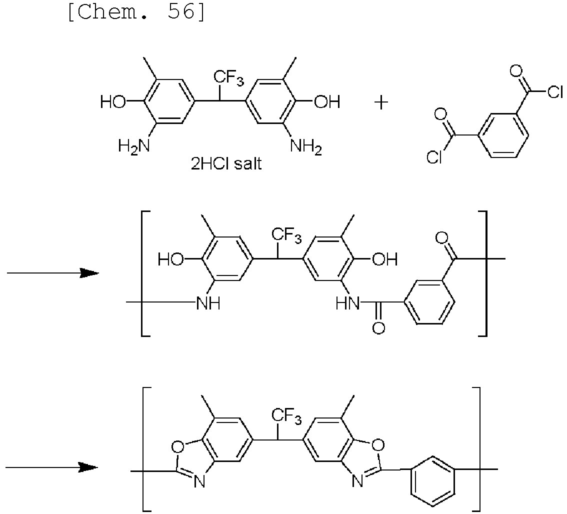

### [Example 8]

Into a 500 mL three-necked flask equipped with a nitrogen inlet tube and a stirrer under ice cooling, 2.8 g (10 mmol) of 2,2-bis(3-amino-4-hydroxyphenyl)-1,1,1-trifluoroethane (BIS-5-FAP-EF), represented by the following formula, and 2.0 g (10 mmol) of IPC were charged, and 1.8 g (23 mmol) of pyridine as a base and 60 g of NMP as an organic solvent were added thereto. Under a nitrogen atmosphere, the mixture was stirred at room temperature (25°C) for 5 hours to obtain a reaction solution. Thereafter, the reaction solution was filtered under pressure to prepare a solution of polyhydroxyamide. As a result of GPC measurement of the solution, Mw was 50057 and Mw/Mn was 2.3.

The prepared solution of polyhydroxyamide was applied onto a glass substrate using a spin coater. Thereafter, under a nitrogen atmosphere, heating was performed at a temperature of 110°C for 10 minutes as a drying step, and then heating at 200°C for 30 minutes and at 300°C for 2 hours was performed as a curing step to obtain a film on the glass substrate. Thereafter, the film was cooled to room temperature, and peeled off from the glass substrate to obtain a film-like cured film (insulating material) . In a case where a film thickness of the cured film was measured, it was 17 um.

From FT-IR measurement results of the cured film, there was absorption specific to oxazole at 1030 to 1100 cm⁻¹, and it was confirmed that the cured film contained polybenzoxazole.

For reference, starting materials and reaction schemes are shown below.

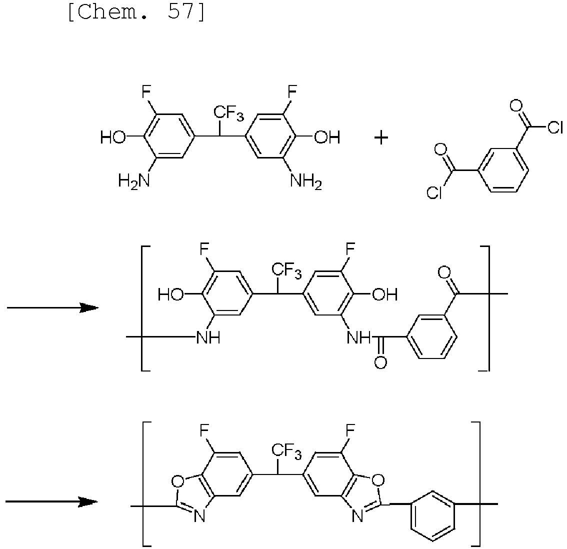

### [Example 9]

Into a 500 mL three-necked flask equipped with a nitrogen inlet tube and a stirrer under ice cooling, 6.0 g (20 mmol) of 2,2-bis(3-amino-4-hydroxyphenyl)-1,1,1-trifluoroethane (BIS-AP-EF), represented by the following formula, 3.0 g (10 mmol) of OBBC, and 2.0 g (10 mmol) of terephthaloyl chloride (hereinafter, may be referred to as TPC) were charged, and 3.6 g (46 mmol) of pyridine as a base and 75 g of NMP as an organic solvent were added thereto. Under a nitrogen atmosphere, the mixture was stirred at room temperature (25°C) for 5 hours to obtain a reaction solution. Thereafter, the reaction solution was filtered under pressure to prepare a solution of polyhydroxyamide. As a result of GPC measurement of the solution, Mw was 30917 and Mw/Mn was 2.4.

The prepared solution of polyhydroxyamide was applied onto a glass substrate using a spin coater. Thereafter, under a nitrogen atmosphere, heating was performed at a temperature of 110°C for 10 minutes as a drying step, and then heating at 200°C for 30 minutes and at 300°C for 2 hours was performed as a curing step to obtain a film on the glass substrate. Thereafter, the film was cooled to room temperature, and peeled off from the glass substrate to obtain a film-like cured film (insulating material) made of polybenzoxazole. In a case where a film thickness of the cured film was measured, it was 26 um.

From FT-IR measurement results, there was absorption specific to oxazole at 1030 to 1100 cm⁻¹. That is, it was confirmed that the cured film contained polybenzoxazole.

For reference, starting materials and reaction schemes are shown below.

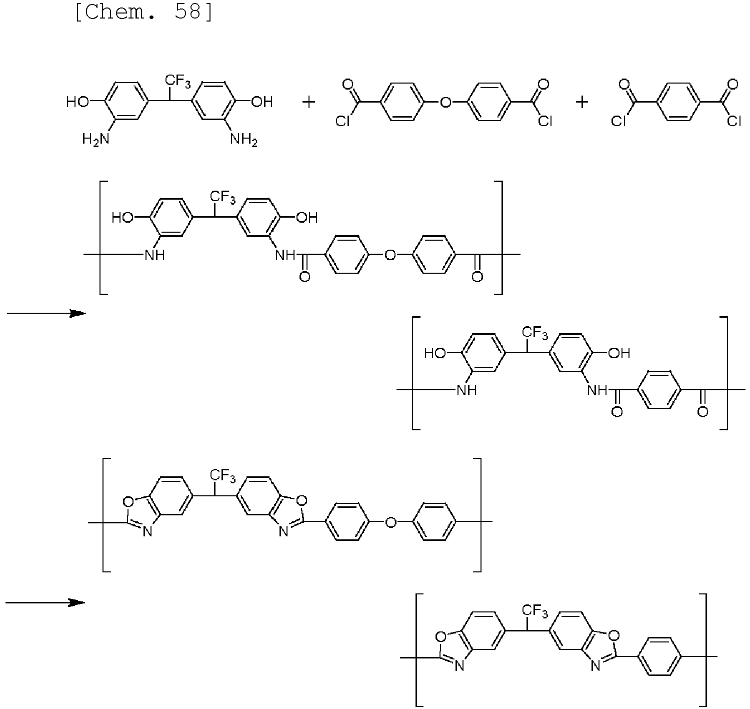

### [Comparative Example 1]

A 25 µm-thick polyimide film (Kapton 100H/V, containing no fluorine atom) manufactured by DU PONT-TORAY CO.,LTD. was prepared as an evaluation sample.

### <Performance evaluation>

### [Dielectric properties]

Using the film-like cured film (insulating material) obtained in Examples and Comparative Examples as an evaluation sample, dielectric properties were evaluated under the conditions and apparatus described in [Dielectric properties] above. Table 1 shows the evaluation results of Examples 1, 2, 5 and Comparative Example 1.

**[Table 1]**

| | Relative permittivity (εr) | Dielectric loss tangent (tan δ) |
|---|---|---|
| Example 1 | 2.8 | 0.0067 |
| Example 2 | 3.2 | 0.0104 |
| Example 5 | 3.0 | 0.0112 |
| Comparative Example 1 | 3.3 | 0.0120 |

The cured films (insulating materials) of Examples were superior to the polyimide film of Comparative Example 1 in dielectric constant and dielectric loss tangent at a frequency condition of 28 GHz. From this result, it is understood that the cured films (insulating materials) of Examples are preferably used for producing a high-frequency electronic component provided in 5G communication equipment.

### [Heat resistance]

Using the film-like cured film (insulating material) obtained in Examples as an evaluation sample, 5%-weight-loss temperature (Td₅) was evaluated under the conditions and apparatus described in [Heat resistance] above. Table 2 shows the evaluation results.

**[Table 2]**

| | 5%-weight-loss temperature |
|---|---|
| Example 1 | 475°C |
| Example 2 | 441°C |
| Example 3 | 440°C |
| Example 6 | 518°C |
| Example 7 | 425°C |
| Example 8 | 475°C |

From Table 2, it is understood that the cured films (insulating materials) of Examples are excellent in heat resistance.

### [Curing temperature]

### (Examples)

The solution of polyhydroxyamide, obtained in Example 1, was applied onto a glass substrate using a spin coater. Thereafter, by heating at a temperature of 110°C for 10 minutes under a nitrogen atmosphere (drying step), a polyhydroxyamide film (not ring-closed) was obtained on the glass substrate.

A curing temperature of the obtained polyhydroxyamide was 275°C.

### (Comparative Example: Measurement and the like of curing temperature of polyamide having -C(CF₃)₂- structure)

Polyhydroxyamide and polybenzoxazole were obtained in the same manner as in Example 1 (the reaction scheme is shown below), except that 2,2-bis(3-amino-4-hydroxyphenyl)-1,1,1,3,3,3-hexafluoropropane (BIS-AP-AF) was used instead of 2,2-bis(3-amino-4-hydroxyphenyl)-1,1,1-trifluoroethane (BIS-AP-EF) .

A curing temperature of the obtained polyhydroxyamide was 289°C.

For reference, the obtained cured film of polybenzoxazole had a dielectric constant of 2.9 and a dielectric loss tangent of 0.0055.

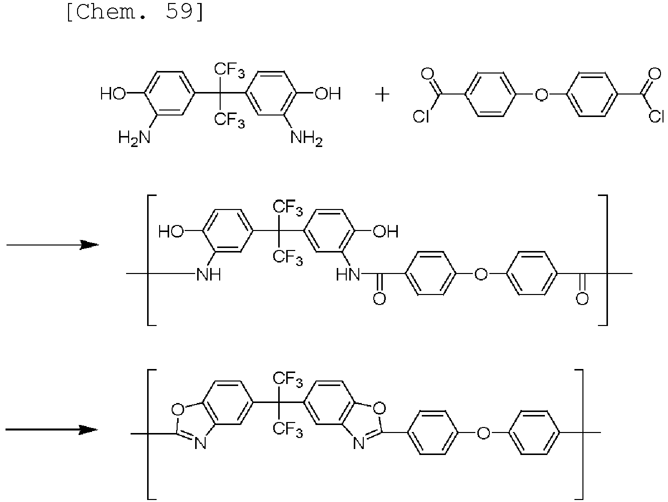

As is clear from the above-described comparison of curing temperatures, the polybenzoxazole having a "-CH(CF₃)-" structure can be cured at a lower temperature than the polybenzoxazole having a "-C(CF₃)₂-" structure. In addition, it is understood that the polybenzoxazole having a "-CH(CF₃)-" structure exhibits equally excellent low dielectric properties, despite having fewer fluorine atoms than the polybenzoxazole having a "-C(CF₃)₂-" structure.

Priority is claimed on Japanese Patent Application No. 2020-119001, filed July 10, 2020, the disclosure of which is incorporated herein by reference.

## Claims

1. A polybenzoxazole comprising:
a structural unit represented by General Formula [1], 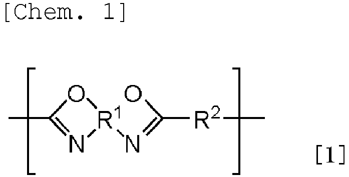 in General Formula [1],
R¹ is a tetravalent organic group represented by General Formula [2], and
R² is a divalent organic group, 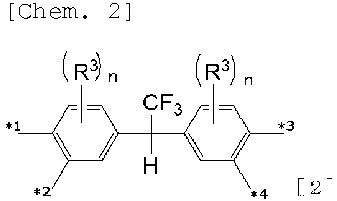 in General Formula [2],
two n's are each independently an integer of 0 to 3,
in a case where a plurality of R³'s are present, the plurality of R³'s each independently represent a monovalent substituent, and
*1, *2, *3, and *4 each independently represent a bonding site, in which one of *1 and *2 is bonded to an oxygen atom in General Formula [1] and the other is bonded to a nitrogen atom in General Formula [1], and one of *3 and *4 is bonded to an oxygen atom in General Formula [1] and the other is bonded to a nitrogen atom in General Formula [1] .

2. The polybenzoxazole according to claim 1,
wherein R¹ is at least one tetravalent organic group selected from the following,

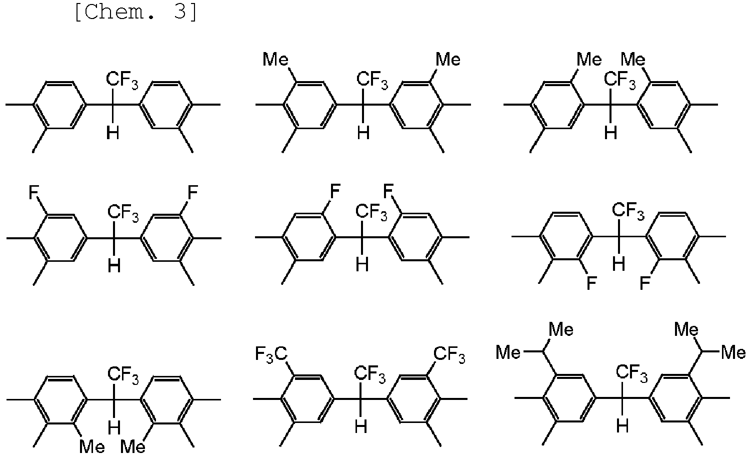

3. The polybenzoxazole according to claim 1 or 2,
wherein R² is at least one divalent organic group selected from the following,

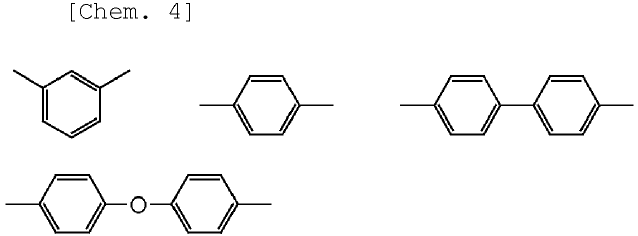

4. A polyamide comprising:
a structural unit represented by General Formula [1A], 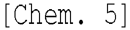 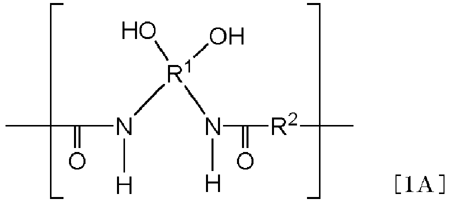 in General Formula [1A],
R¹ represents a tetravalent organic group represented by General Formula [2], and
R² represents a divalent organic group, 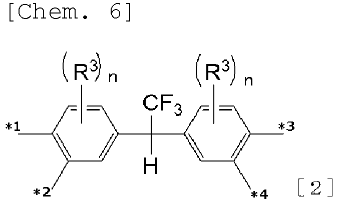 in General Formula [2],
two n's are each independently an integer of 0 to 3,
in a case where a plurality of R³'s are present, the plurality of R³'s each independently represent a monovalent substituent, and
*1, *2, *3, and *4 each independently represent a bonding site, in which one of *1 and *2 is bonded to an oxygen atom in a hydroxy group of General Formula [1A] and the other is bonded to a nitrogen atom in General Formula [1A], and one of *3 and *4 is bonded to an oxygen atom in a hydroxy group of General Formula [1A] and the other is bonded to a nitrogen atom in General Formula [1A].

5. The polyamide according to claim 4,
wherein R¹ is at least one tetravalent organic group selected from the following,

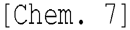

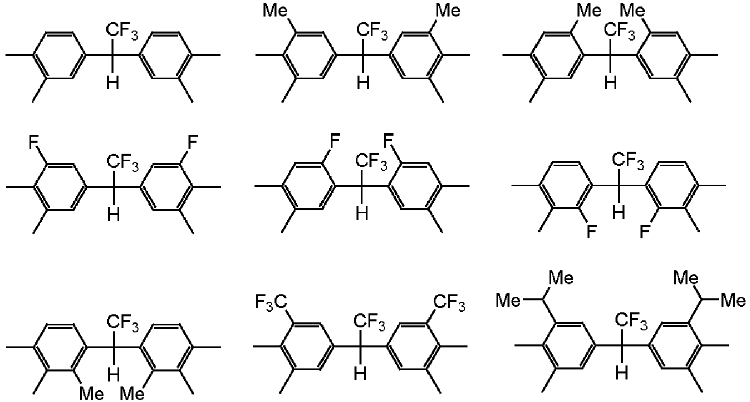

6. The polyamide according to claim 4 or 5,
wherein R² is at least one divalent organic group selected from the following,

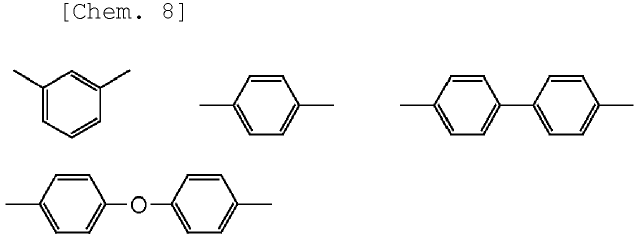

7. The polyamide according to any one of claims 4 to 6,
wherein a weight-average molecular weight is 1,000 or more and 1,000,000 or less.

8. A polyamide solution comprising:
the polyamide according to any one of claims 4 to 7; and
an organic solvent.

9. The polyamide solution according to claim 8,
wherein the organic solvent includes at least one selected from the group consisting of an amide-based solvent, an ether-based solvent, an aromatic solvent, a halogen-based solvent, and a lactone-based solvent.

10. The polyamide solution according to claim 8 or 9,
wherein a concentration of the polyamide is 0.1% by mass or more and 50% by mass or less.

11. An insulating material for a high-frequency electronic component, comprising:
the polybenzoxazole according to any one of claims 1 to 3.

12. The insulating material for a high-frequency electronic component according to claim 11,
wherein a 5%-weight-loss temperature Td₅ is 400°C or higher.

13. The insulating material for a high-frequency electronic component according to claim 11 or 12,
wherein a dielectric loss tangent at a frequency of 28 GHz is 0.012 or less.

14. The insulating material for a high-frequency electronic component according to any one of claims 11 to 13,
wherein a relative permittivity at a frequency of 28 GHz is 3.2 or less.

15. A high-frequency electronic component comprising:
the insulating material for a high-frequency electronic component according to any one of claims 11 to 14.

16. A high-frequency equipment comprising:
the high-frequency electronic component according to claim 15.

17. An insulating material for producing a high-frequency electronic component, comprising:
the polyamide according to any one of claims 4 to 7.

18. The insulating material for producing a high-frequency electronic component according to claim 17,
wherein a weight-average molecular weight of the polyamide is 500,000 or less.

19. A method for producing the polyamide according to any one of claims 4 to 7, the method comprising:
a step of polycondensing a diamine represented by General Formula [DA] or a salt of the diamine with a dicarboxylic acid represented by General Formula [DC1] or [DC2] or a derivative of the dicarboxylic acid, 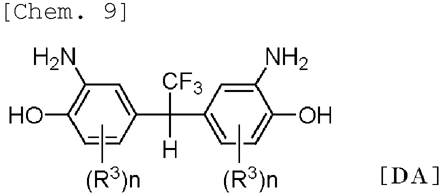
in General Formula [DA], n and R³ are the same as n and R³ in General Formula [2], 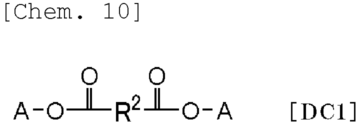
in General Formula [DC1], R² is the same as R² in General Formula [1A], and two A's are each independently a hydrogen atom, an alkyl group having 1 to 10 carbon atoms, or an aromatic hydrocarbon group having 6 to 10 carbon atoms, 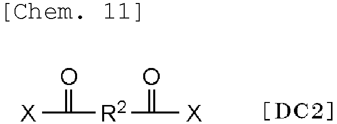
in General Formula [DC2], R² is the same as R² in General Formula [1A], and two X's are each independently a fluorine atom, a chlorine atom, a bromine atom, an iodine atom, or an active ester group.

20. A method for producing the polybenzoxazole according to any one of claims 1 to 3, the method comprising:
a first step of producing a polyamide by the method for producing the polyamide according to claim 19; and
a second step of dehydrating and ring-closing the polyamide obtained in the first step.

21. A method for producing an insulating material for a high-frequency electronic component, the method comprising:
a coating step of applying the polyamide solution according to any one of claims 8 to 10 onto a supporting base material;
a drying step of drying the solvent contained in the applied polyamide solution to obtain a resin film including the polyamide; and
a heating step of heat-treating the resin film to form a cured film.

22. The method for producing an insulating material for a high-frequency electronic component according to claim 21,
wherein the supporting base material is at least one selected from the group consisting of glass, a silicon wafer, stainless steel, alumina, copper, nickel, polyethylene terephthalate, polyethylene glycol terephthalate, polyethylene glycol naphthalate, polycarbonate, polyimide, polyamide-imide, polyetherimide, polyetheretherketone, polypropylene, polyether sulfone, polyethylene terephthalate, polyphenylene sulfone, and polyphenylene sulfide.

23. The method for producing an insulating material for a high-frequency electronic component according to claim 21 or 22,
wherein a film thickness of the cured film is 1 pm or more and 1000 um or less.

24. The method for producing an insulating material for a high-frequency electronic component according to any one of claims 21 to 23,
wherein the drying step is performed at a temperature of 50°C or higher and 250°C or lower.

25. The method for producing an insulating material for a high-frequency electronic component according to any one of claims 21 to 24,
wherein the curing step is performed at a temperature of 150°C or higher and 400°C or lower.

26. A diamine represented by General Formula [DA] or a salt of the diamine, 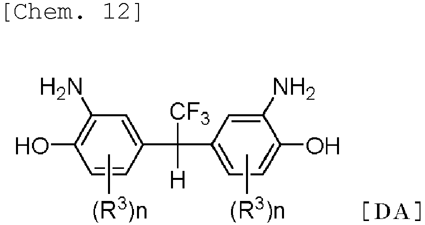 in General Formula [DA], two n's are each independently an integer of 0 to 3, and in a case where a plurality of R³' s are present, the plurality of R³'s each independently represent a monovalent substituent.

27. The diamine or the salt of the diamine according to claim 26,
wherein a diamine represented by Formula [DA-1] or a salt of the diamine is excluded,

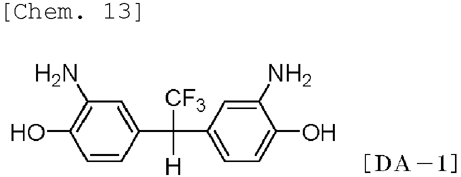

28. The diamine or the salt of the diamine according to claim 26 or 27,
wherein the diamine or the salt of the diamine is represented by General Formula [DA-2], 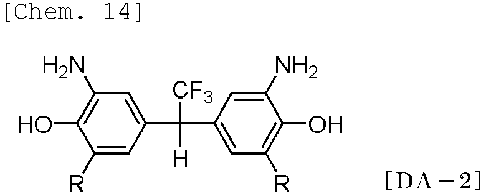
in General Formula [DA-2], two R's each independently represent an alkyl group having 1 to 6 carbon atoms or a halogen atom.

29. The diamine or the salt of the diamine according to claim 26 or 27,
wherein the diamine or the salt of the diamine is represented by General Formula [DA-3], 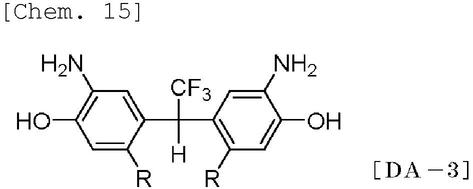
in General Formula [DA-3], two R's each independently represent an alkyl group having 1 to 6 carbon atoms or a halogen atom.

30. The diamine or the salt of the diamine according to any one of claims 26 to 28,
wherein the diamine or the salt of the diamine is represented by General Formula [DA-4],

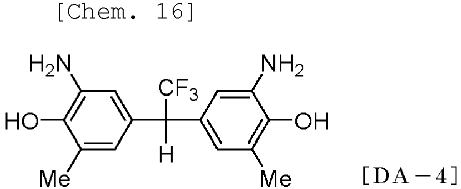

31. The diamine or the salt of the diamine according to any one of claims 26 to 28,
wherein the diamine or the salt of the diamine is represented by General Formula [DA-5],

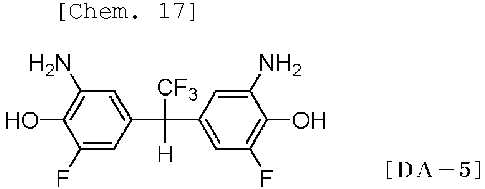

32. The diamine or the salt of the diamine according to any one of claims 26 to 28,
wherein the diamine or the salt of the diamine is represented by General Formula [DA-6],

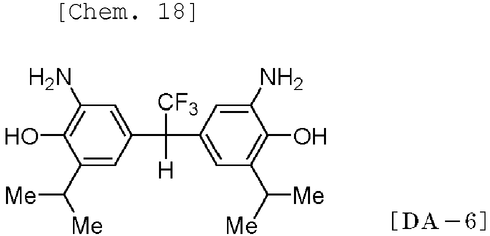

33. The diamine or the salt of the diamine according to claim 26, 27, or 29,
wherein the diamine or the salt of the diamine is represented by General Formula [DA-7],

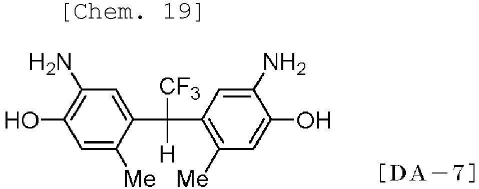

34. The diamine or the salt of the diamine according to any one of claims 26 to 33,
wherein a fluoride ion concentration is 5 ppm or less.

35. A method for producing the diamine or the salt of the diamine according to any one of claims 26 to 34, the method comprising:
a step of reducing a bisnitrophenol represented by General Formula [DN], 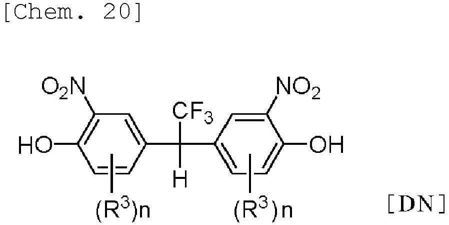
in General Formula [DN], definitions of two n's and R³ are the same as definitions of two n's and R³ in General Formula [DA].
